(19) Europäisches Patentamt
European Patent Office
Office européen des brevets

(11) EP 1 968 951 B1

(12) **EUROPEAN PATENT SPECIFICATION**

(45) Date of publication and mention of the grant of the patent:
**22.06.2011 Bulletin 2011/25**

(21) Application number: **06835834.0**

(22) Date of filing: **11.12.2006**

(51) Int Cl.:
***C07D 241/22*** *(2006.01)* ***A61K 31/4965*** *(2006.01)*
***A61P 11/00*** *(2006.01)* ***A61P 11/06*** *(2006.01)*

(86) International application number:
**PCT/SE2006/001409**

(87) International publication number:
**WO 2007/069978 (21.06.2007 Gazette 2007/25)**

(54) **NOVEL N-(FLUORO-PYRAZINYL)-PHENYLSULFONAMID.ES AS MOODULATORS OF CHEMOKINE RECEPTOR CCR4.**

NEUE N-(FLUORPYRAZINYL)PHENYLSULFONAMIDE ALS MODULATOREN DES CHEMOKINREZEPTORS CCR4

NOUVEAUX N-(FLUORO-PYRAZINYL)PHENYLSULFONAMIDES UTILISES COMME MODULATEURS DU RECEPTEUR DE LA CHIMIOKINE CCR4

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **12.12.2005 SE 0502733**

(43) Date of publication of application:
**17.09.2008 Bulletin 2008/38**

(73) Proprietor: **AstraZeneca AB**
**151 85 Södertälje (SE)**

(72) Inventors:
- **CHESHIRE, David**
  **Leicestershire LE11 5RE (GB)**
- **KINDON, Nicholas**
  **Leicestershire LE11 5RH (GB)**
- **METE, Antonio**
  **Leicestershire LE11 5RH (GB)**
- **ROBERTS, Bryan**
  **Leicestershire LE11 5RH (GB)**

(56) References cited:
**WO-A1-03/004472** **WO-A1-03/059893**
**WO-A1-2004/108692** **WO-A1-2004/108717**
**WO-A1-2005/021513** **GB-A- 928 151**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to N-(fluoro-pyrazinyl)-phenylsulfonamides, processes and intermediates used in their preparation, pharmaceutical compositions containing them and their use in therapy.

Chemokines play an important role in immune and inflammatory responses in various diseases and disorders, including asthma and allergic diseases, as well as autoimmune pathologies such as rheumatoid arthritis and atherosclerosis. These small-secreted molecules are a growing superfamily of 8-14 kDa proteins characterised by a conserved four cysteine motif. At the present time, the chemokine superfamily comprises three groups exhibiting characteristic structural motifs, the Cys-X-Cys (C-X-C), Cys-Cys (C-C) and Cys-$X_3$-Cys (C-$X_3$-C) families. The C-X-C and C-C families have sequence similarity and are distinguished from one another on the basis of a single amino acid insertion between the NH-proximal pair of cysteine residues. The C-$X_3$-C family is distinguished from the other two families on the basis of having a triple amino acid insertion between the NH-proximal pair of cysteine residues.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils. Examples include human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β), Thymus and Activation Regulated Chemokine (TARC, CCL17) and Macrophage Derived Chemokine (MDC, CCL22). The C-$X_3$-C chemokine (also known as fractalkine) is a potent chemoattractant and activator of microglia in the central nervous system (CNS) as well as of monocytes, T cells, NK cells and mast cells.

Studies have demonstrated that the actions of chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and $CX_3CR1$ for the C-$X_3$-C family. These receptors represent good targets for drug development since agents which modulate these receptors would be useful in the treatment of disorders and diseases such as those mentioned above. Agents effective at modulating the CCR4 receptor are of particular interest for use in the treatment of inflammatory diseases.

WO 03/051870 and WO 03/059893 disclose a series of sulphonamide compounds said to be useful for treating various diseases. It has now surprisingly been found that a narrow class of compounds generically disclosed in WO 03/059893 exhibit advantageous pharmaceutical properties. For example, in addition to high potency the compounds of the present invention also exhibit low plasma protein binding to human plasma, which increases effectiveness in vivo.

The present invention provides a compound of formula (I) or a pharmaceutically acceptable salt thereof:

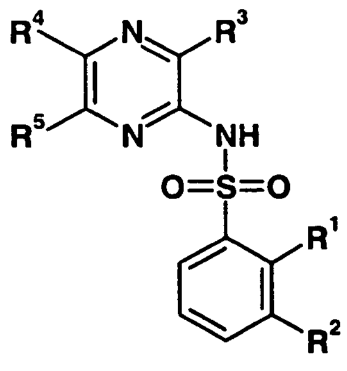

(I)

wherein

$R^1$ is selected from methyl, chlorine and fluorine;

$R^2$ is selected from methyl, chlorine and fluorine;

$R^3$ is methoxy;

one of $R^4$ and $R^5$ is fluorine and the other of $R^4$and $R^5$ is selected from hydrogen and hydroxymethyl.

Suitable pharmaceutically acceptable salts of formula (I) include include metal salts, such as an alkali metal salt (for example a sodium or potassium salt) or an alkaline earth metal salt (for example magnesium or calcium), or an organic amine salt for example ammonia, triethylamine, piperidine, piperazine or dibenzylamine.

It will be understood that certain compounds of the present invention and pharmaceutically acceptable salts thereof may exist in solvated, for example hydrated, as well as unsolvated forms. It is to be understood that the present

invention encompasses all such solvated forms. The present invention also encompasses any tautomers of compounds of formula (I), or mixtures thereof.

In an embodiment of the invention, $R^1$ is selected from chlorine and fluorine, and $R^2$ is selected from chlorine and fluorine.

In an embodiment of the invention, $R^1$ is chlorine and $R^2$ is chlorine.

In an embodiment of the invention, $R^4$ is fluorine and $R^5$ is selected from hydrogen and hydroxymethyl.

In an embodiment of the invention, $R^5$ is fluorine and $R^4$ is selected from hydrogen and hydroxymethyl.

In an embodiment of the invention, one of $R^4$ and $R^5$ is fluorine and the other of $R^4$ and $R^5$ is hydrogen.

In an embodiment of the invention, one of $R^4$ and $R^5$ is fluorine and the other of $R^4$ and $R^5$ is hydroxymethyl.

In an embodiment of the invention, $R^4$ is hydrogen and $R^5$ is fluorine.

In an embodiment of the invention, $R^4$ is hydroxymethyl and $R^5$ is fluorine.

In an embodiment of the invention, $R^4$ is fluorine and $R^5$ is hydrogen.

In an embodiment of the invention, $R^4$ is fluorine and $R^5$ is hydroxymethyl.

In an embodiment of the present invention the compound of formula (I) is selected from:

2-Chloro-3-fluoro-*N*-(5-fluoro-3-methoxypyrazin-2-yl)- benzenesulfonamide,
2,3-Dichloro-*N*-(5-fluoro-3-methoxypyrazin-2-yl)-benzenesulfonamide,
2,3-Dichloro-*N*-(6-fluoro-3-methoxypyrazin-2-yl)-benzenesulfonamide,
2,3-Dichloro-*N*-[6-fluoro-5-(hydroxymethyl)-3-methoxypyrazin-2-yl]-benzenes ulfonamide,
3-Chloro-2-fluoro-*N*-(5-fluoro-3-methoxypyrazin-2-yl)-benzenesulfonamide,
2,3-Dichloro-*N*-[5-fluoro-6-(hydroxymethyl)-3-methoxypyrazin-2-yl]-benzenesulfonamide,
3-Chloro-*N*-(5-fluoro-3-methoxypyrazin-2-yl)-2-methyl-benzenesulfonamide,

or a pharmaceutically acceptable salt thereof.

Pharmaceutical compounds may be metabolised to form other compounds in vivo. For N-pyrazinyl-phenyl sulphonamides, one type of metabolite that may be formed in vivo is an aminopyrazine derivative. Some aminopyrazine derivatives display mutagenicity, i.e. they are AMES +ve according to the test procedure of Maron and Ames described in Mutation Res. 1983; 113:173-215. It is a further advantage of the compounds of the present invention that their aminopyrazine derivatives are not mutagenic.

According to the present invention there is also provided a process for the preparation of a compound of formula (I) or a pharmaceutically acceptable salt thereof, which comprises

(a) reacting a compound of formula (II), wherein $R^1$, $R^2$ and $R^3$ are as defined in formula (I) and one of $R^6$ and $R^7$ is hydrogen and the other of $R^6$ and $R^7$ is $NH_2$, with a nitrite salt in the presence of a fluorinating agent,

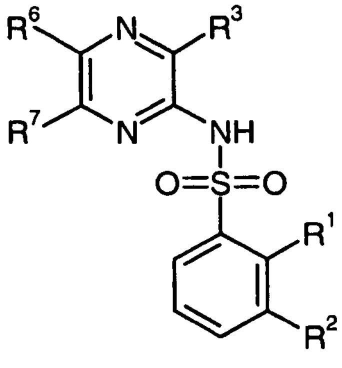
(II),

or

(b) reacting a compound of formula (III), wherein $R^1$, $R^2$ and $R^3$ are as defined in formula (I) and one of $R^8$ and $R^9$ is fluorine and the other of $R^8$ and $R^9$ is bromine, with hydrogen in the presence of a palladium catalyst,

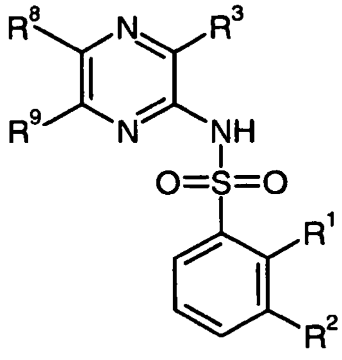

(III),

or

(c) where one of $R^4$ and $R^5$ is hydroxymethyl, reacting a compound of formula (III) as described in (b), with carbon monoxide in the presence of a palladium catalyst, and subsequently treating the resulting acid (or $C_{1-4}$ alkyl ester thereof) with a suitable reducing agent, or

(d) where one of $R^4$ and $R^5$ is fluorine and the other of $R^4$ and $R^5$ is hydrogen, reacting a compound of formula (IV), wherein $R^3$ is as defined in formula (I) and where one of $R^{10}$ and $R^{11}$ is fluorine and the other of $R^{10}$ and $R^{11}$ is hydrogen,

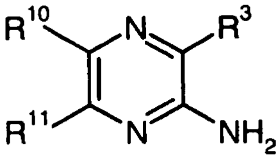

(IV),

with a compound of formula (V), wherein $R^1$ and $R^2$ are as defined in formula (I)

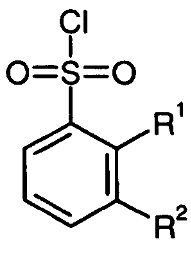

(V)

or

(e) where $R^2$ is fluorine and $R^1$ is chlorine, reacting a compound of formula (VI) wherein $R^3$, $R^4$ and $R^5$ are as defined in formula (I), with hexachloroethane in the presence of a lithium amide or alkyl lithium base,

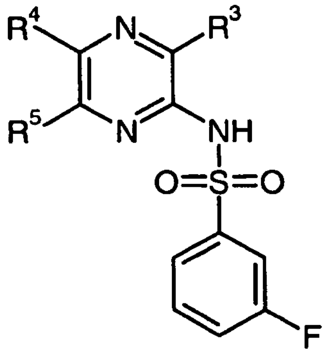
(VI),

and optionally after (a), (b), (c), (d) or (e) carrying out one or more of the following:

- converting the compound to a further compound of the invention or
- forming a pharmaceutically acceptable salt of the compound.

It will be understood by those skilled in the art that in compounds of formula (II) the hydrogen atom that is located at $R^6$ or $R^7$ will not undergo transformation in process (a) and will be the hydrogen atom at either $R^4$ or $R^5$ in the resulting compound of formula (I). Similarly, in compounds of formula (III) the fluorine atom that is located at $R^8$ or $R^9$ will not undergo transformation in process (b) and will be the fluorine atom at either $R^4$ or $R^5$ in the resulting compound of formula (I). In compounds of formula (IV), the substituents at $R^{10}$ and $R^{11}$ will not undergo transformation in process (d) and they correspond directly with the substituents at $R^4$ or $R^5$ in the resulting compound of formula (I).

In process (a) the reaction may be performed in a solvent such as acetonitrile, at a temperature in the range of - 10°C to 50 °C. The nitrite salt may be sodium nitrite (either in the form of an aqueous solution or solid) and the fluorinating agent may for example be tetrafluoroboric acid or hydrogen-fluoride in pyridine.

In process (b) the reaction may be performed in a suitable solvent such as ethyl acetate at a hydrogen pressure of, for example, 1 bar, in the presence of a suitable base such as triethylamine and a palladium catalyst such as 5% Pd on charcoal, at a temperature in the range of 0 to 50 °C.

In process (c) the initial reaction may be performed in a suitable solvent such as methanol, ethanol at a carbon monoxide pressure of, for example, 3-7 bar, in the presence of a suitable tertiary amine base such as triethylamine or diisopropylethylamine and a suitable palladium catalyst such as dichloro[1,1'-bis(diphenylphosphino)ferrocene]palladium (II) dichloromethane adduct, and at a temperature in the range of 70 to 100°C. When performed in the presence of an alcohol solvent the resulting acid will be converted to the corresponding alkyl ester, e.g. the methyl ester will be formed when methanol is the solvent. The reaction may also be performed in a solvent such as dimethylformamide, in which case the acid will be obtained. Subsequent reduction of the alkyl ester to the alcohol may be performed in a suitable solvent such as tetrahydrofuran using a suitable reducing agent such as lithium triethylborohydride at a temperature in the range of 0 to 30°C. Reduction of the acid to the alcohol may be achieved using conventional chemistry.

In process (d) the reaction may be performed in a suitable solvent such as 1,2-dimethoxyethane or tetrahydrofuran, at a temperature in the range of 0 to 50 °C, under the influence of a base such as NaH or potassium tert-butoxide.

In process (e) the reaction may be performed in suitable solvent such as tetrahydrofuran or hexane or mixtures thereof, by treatment with a suitable base such as lithium diisopropylamide, followed by the addition of hexachloroethane, at a temperature in the range of -78 to 0°C.

Compounds of formulae (II), (III) or (V) are either commercially available, are known in the literature or may be prepared using known techniques. Examples of preparation methods for certain of these compounds are given hereinafter in the examples. Other examples can be prepared by analogous methods.

For example, compounds of formula (II) wherein $R^6$ is $NH_2$ and $R^7$ is hydrogen may be prepared according to Scheme 1, wherein $R^1$, $R^2$ and $R^3$ are as defined in formula (I).

**Scheme 1**

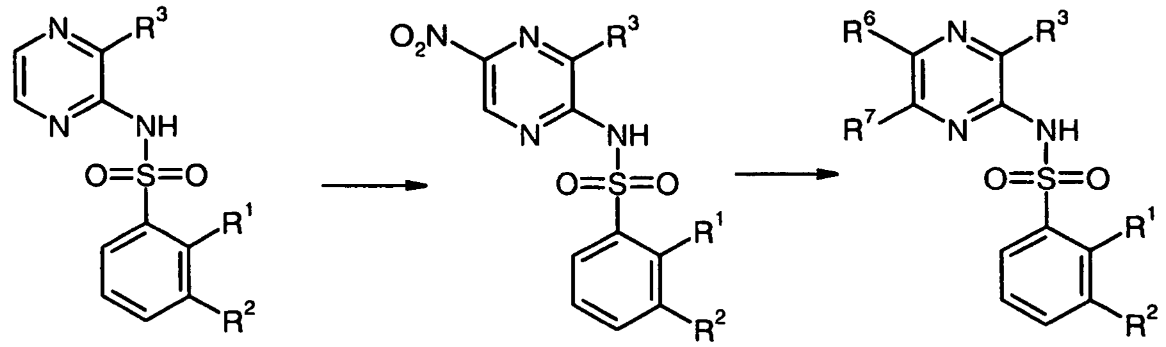

(VII) (VIII)

(II)

According to Scheme 1, compounds of formula (VII) are converted to compounds of formula (VIII) by reacting (VII) with fuming nitric acid in a suitable solvent such as acetic acid at a temperature of from 50 to 100°C, or alternatively reacting (VII) with nitronium tetrafluoroborate in a suitable solvent such as acetonitrile or sulfolane at a temperature of from 0 to 50°C. Subsequently, (VIII) is converted to a compound of formula (II) wherein $R^6$ is $NH_2$ and $R^7$ is hydrogen, by hydrogenation (1-3 bar) in a suitable solvent such as acetic acid or acetic acid/ethyl acetate mixtures with a suitable hydrogenation catalyst such as 5-10% palladium on charcoal at a temperature of from 20 to 70°C, or alternatively by reacting (VIII) with a metal such as iron powder in a suitable solvent such as ethyl acetate containing concentrated hydrochloric acid heated at a temperature of from 50 to 100°C. Compounds of formula (VII) may be prepared by methods according or analogous to those described in WO03/059893.

Alternatively, compounds of formula (II) may be prepared according to Scheme 2, wherein $R^1$, $R^2$ and $R^3$ are as defined in formula (I).

**Scheme 2**

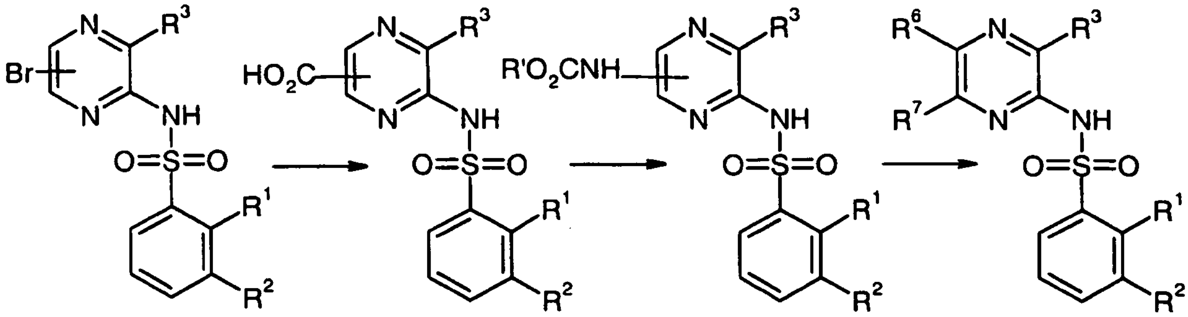

(IX) (X)

(XI) (II)

According to Scheme 2, (IX) is converted to (X) by reacting (IX) with carbon monoxide (3-7 bar) in a suitable solvent such as methanol in the presence of a suitable tertiary amine base such as triethylamine or diisopropylethylamine and suitable palladium catalyst such as dichloro[1,1'-bis(diphenylphosphino)ferrocene]palladium(II) dichloromethane at a temperature of from 60 to 100°C, followed by hydrolysis of the methyl ester to yield (X). (X) is converted to carbamate (XI) by reacting (X) with diphenylphosphoryl azide and para methoxybenzyl alcohol or tertiary butanol, in the presence of a suitable amine base such as triethylamine in a suitable solvent such as tetrahydrofuran heated to reflux. Carbamate (XI) is converted to (II) by treatment with a suitable acid such as HCl (e.g. 4M) in dioxane. Compounds of formula (IX) may be prepared by methods according or analogous to those described in WO03/059893.

Compounds of formula (III) may, for example, be prepared by as depicted in Scheme 3, wherein $R^1$, $R^2$ and $R^3$ are as defined in formula (I).

**Scheme 3**

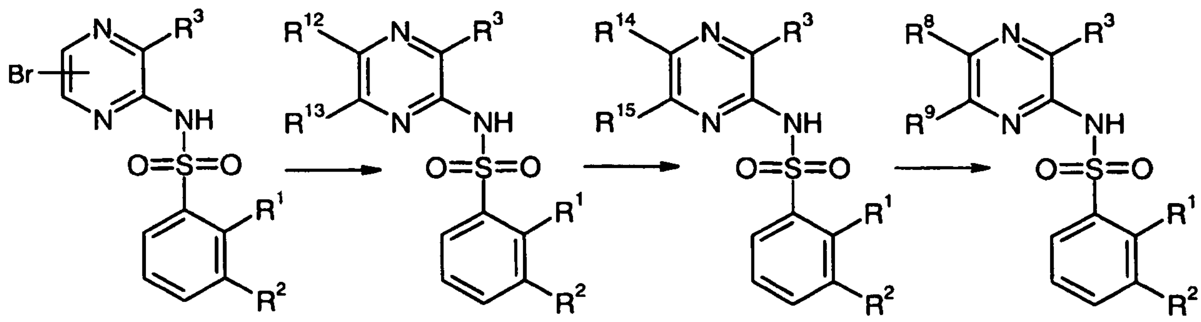

(XII) (XIII)

(XIV) (III)

According to Scheme 3, compounds of formula (XII) are converted to compounds of formula (XIII), wherein one of $R^{12}$ and $R^{13}$ is $NO_2$ and the other of $R^{12}$ and $R^{13}$ is bromine, by reacting (XII) with fuming nitric acid in a suitable solvent such as acetic acid at a temperature of from 50 to 100°C, or alternatively reacting (XII) with nitronium tetrafluoroborate in a suitable solvent such as acetonitrile or sulfolane at a temperature of from 0 to 50 °C. Subsequently, (XIII) is converted to (XIV), wherein one of $R^{14}$ and $R^{15}$ is $NH_2$ and the other of $R^{14}$ and $R^{15}$ is bromine, by hydrogenation (1-3 bar) in a suitable solvent such as acetic acid or acetic acid/ethyl acetate mixtures with a suitable hydrogenation catalyst such as 5-10% palladium on charcoal at a temperature of from 20 to 70°C, or alternatively by treating (XIII) with a metal such as iron powder in a suitable solvent such as ethyl acetate containing concentrated hydrochloric acid heated at a temperature of from 50 to 100°C. (XIV) may then be converted into (III) by reacting (XIV) with a nitrite salt in the presence of fluorinating agent in an analogous method to that described in process (a) herein above. Compounds of formula (XII) may prepared by methods according or analogous to those described in WO03/059893.

Compounds of formula (IV) wherein $R^{10}$ is fluorine and $R^{11}$ is hydrogen may be prepared as depicted in Scheme 4.

**Scheme 4**

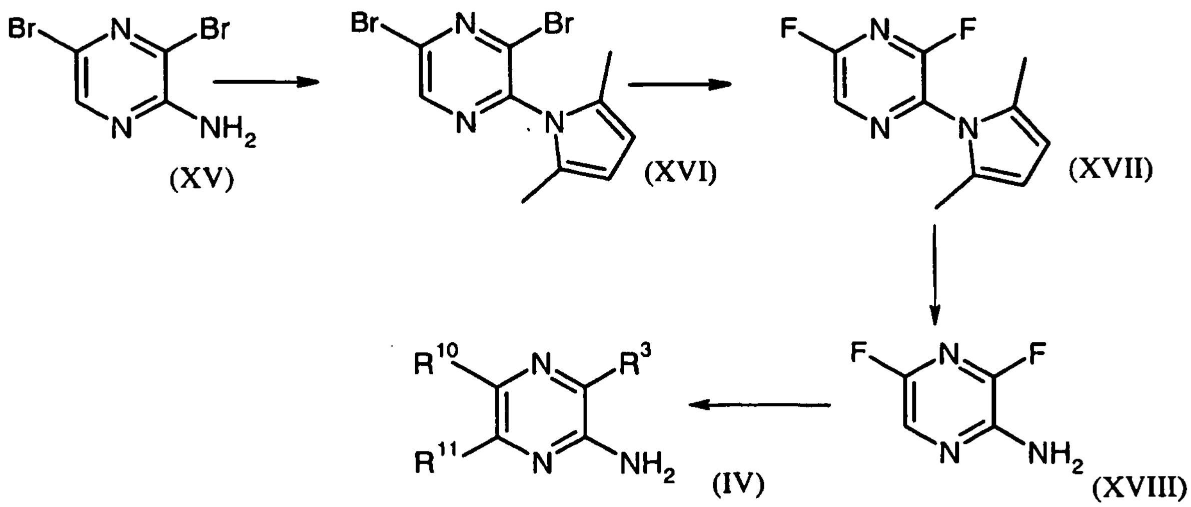

(XV) (XVI) (XVII)

(IV) (XVIII)

According to Scheme 4, compound (XV) is converted to (XVI) by reacting (XV) with acetonylacetone in the presence of para toluene sulphonic acid in a suitable solvent such as toluene at a temperature of from 80 to 110°C.

(XVI) is then converted to (XVII) by reaction of (XVI) with potassium fluoride in the presence of 18-crown-6 in a suitable solvent such as 2-methoxyethyl ether at a temperature of from 100 to 130°C. Treating (XVII) with hydrochloric acid in water and a suitable solvent such as dioxane at a temperature of from 40 to 60°C yields (XVIII), which is converted to a compound of formula (IV), wherein $R^{10}$ is fluorine and $R^{11}$ is hydrogen, by reacting (XVIII) with sodium methoxide in methanol at a temperature of from 0 to 30 °C. Compounds of formula (IV) wherein $R^{11}$ is fluorine and $R^{10}$ is hydrogen may be prepared by analogous chemistry.

Intermediate compounds of formula (IV) have not been prepared previously. Accordingly, in a further aspect the present invention further provides a compound of formula (IV),

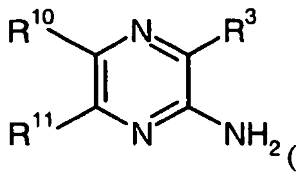
(IV)

wherein $R^3$ is methoxy; one of $R^{10}$ and $R^{11}$ is fluorine and the other of $R^{10}$ and $R^{11}$ is hydrogen. In one embodiment of the invention $R^{10}$ is fluorine and $R^{11}$ is hydrogen. In another embodiment of the invention of $R^{11}$ is fluorine and $R^{10}$ is hydrogen.

Compounds of formula (V) are known in the literature or may be prepared by known methods.

Compounds of formula (VI) may, for example, be prepared by analogous methods to those described herein above for the formation of compounds of formula (I).

It will be appreciated by those skilled in the art that in the processes of the present invention certain functional groups such as hydroxyl, carboxyl or amino groups in the starting reagents or intermediate compounds may need to be protected by protecting groups. Thus, the preparation of the compounds of formula (I) may involve at a certain stage the addition/removal of one or more protecting groups. The protection and deprotection of functional groups is described in Protective Groups in Organic Synthesis', 2nd edition, T.W. Greene and P.G.M. Wuts, Wiley-Interscience (1991) and 'Protecting Groups', P.J. Kocienski, Georg Thieme Verlag (1994).

The compounds of the invention, or pharmaceutically acceptable salts thereof, have activity as pharmaceuticals, in particular as modulators of chemokine receptor (especially CCR4) activity. Diseases and conditions which may be treated with the compounds include:

1. *respiratory tract:* obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
2. *bone and joints:* arthritides associated with or including osteoarthritis/osteoarthrosis, both primary and secondary to, for example, congenital hip dysplasia; cervical and lumbar spondylitis, and low back and neck pain; rheumatoid arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease; primary and secondary Sjogren's syndrome; systemic sclerosis and limited scleroderma; systemic lupus erythematosus, mixed connective tissue disease, and undifferentiated connective tissue disease; inflammatory myopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arteritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndrome, and Familial Hibernian Fever, Kikuchi disease; drug-induced arthalgias, tendonititides, and myopathies;

3. *pain and connective tissue remodelling of musculoskeletal disorders due to injury [for example sports injury] or disease:* arthritides (for example rheumatoid arthritis, osteoarthritis, gout or crystal arthropathy), other joint disease (such as intervertebral disc degeneration or temporomandibular joint degeneration), bone remodelling disease (such as osteoporosis, Paget's disease or osteonecrosis), polychondritits, scleroderma, mixed connective tissue disorder, spondyloarthropathies or periodontal disease (such as periodontitis);

4. *skin:* psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis;cutaneous lymphomas, non-melanoma skin cancer and other dysplastic lesions; drug-induced disorders including fixed drug eruptions;

5. *eyes:* blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral , fungal, and bacterial;

6. *gastrointestinal tract:* glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema);

7. *abdominal:* hepatitis, including autoimmune, alcoholic and viral; fibrosis and cirrhosis of the liver; cholecystitis; pancreatitis, both acute and chronic;

8. *genitourinary:* nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);

9. *allograft rejection:* acute and chronic following, for example, transplantation of kidney, heart, liver, lung, bone marrow, skin or cornea or following blood transfusion; or chronic graft versus host disease;

10. CNS: Alzheimer's disease and other dementing disorders including CJD and nvCJD; amyloidosis; multiple sclerosis and other demyelinating syndromes; cerebral atherosclerosis and vasculitis; temporal arteritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes;

11. other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;

12. other disorders with an inflammatory or immunological component; including acquired immune deficiency syndrome (AIDS), leprosy, Sezary syndrome, and paraneoplastic syndromes;

13. *cardiovascular:* atherosclerosis, affecting the coronary and peripheral circulation; pericarditis; myocarditis , inflammatory and auto-immune cardiomyopathies including myocardial sarcoid; ischaemic reperfusion injuries; endocarditis, valvulitis, and aortitis including infective (for example syphilitic); vasculitides; disorders of the proximal and peripheral veins including phlebitis and thrombosis, including deep vein thrombosis and complications of varicose veins;

14. *oncology:* treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumour recurrences, and paraneoplastic syndromes; and,

15. *gastrointestinal tract:* Coeliac disease, proctitis, eosinopilic gastro-enteritis, mastocytosis, Crohn's disease, ulcerative colitis, microscopic colitis, indeterminant colitis, irritable bowel disorder, irritable bowel syndrome, non-inflammatory diarrhea, food-related allergies which have effects remote from the gut, e.g., migraine, rhinitis and eczema.

Accordingly, the present invention further provides a compound of formula (I), or a pharmaceutically acceptable salt thereof, as hereinbefore defined for use in therapy.

The compounds of the present invention may be used to treat diseases by modulating activity of a CC chemokine

receptor subfamily, in particular, by modulating activity of the CCR4 receptor. Particular conditions which can be treated with the compound of the invention are asthma, rhinitis and inflammatory skin disorders, diseases in which there are raised TARC, MDC or CCR4 levels.

In a further aspect, the present invention provides the use of a compound of formula (I), or a pharmaceutically acceptable salt thereof, as hereinbefore defined in the manufacture of a medicament for use in therapy.

In a still further aspect, the present invention provides the use of a compound of formula (I), or a pharmaceutically acceptable salt thereof, as hereinbefore defined in the manufacture of a medicament for the treatment of human diseases or conditions in which modulation of chemokine receptor activity, particularly CCR4 activity, is beneficial.

In a still further aspect, the present invention provides the use of a compound of formula (I), or a pharmaceutically acceptable salt thereof, as hereinbefore defined in the manufacture of a medicament for the treatment of human diseases or conditions in which modulation of the CCR4 receptor is beneficial.

In a still further aspect, the present invention provides the use of a compound of formula (I), or a pharmaceutically acceptable salt thereof, as hereinbefore defined in the manufacture of a medicament for the treatment of asthma.

In a still further aspect, the present invention provides the use of a compound of formula (I), or a pharmaceutically acceptable salt thereof, as hereinbefore defined in the manufacture of a medicament for the treatment of chronic obstructive pulmonary disease,

In the context of the present specification, the term "therapy" also includes "prophylaxis" unless there are specific indications to the contrary. The terms "therapeutic" and "therapeutically" should be construed accordingly.

For the above-mentioned therapeutic uses the dosage administered will, of course, vary with the compound employed, the mode of administration, the treatment desired and the disorder indicated.

The compound of formula (I) and pharmaceutically acceptable salts thereof may be used on their own but will generally be administered in the form of a pharmaceutical composition in which the formula (I) compound/salt (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt thereof, as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt thereof, as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally. Conveniently the compound of the invention is administered orally.

The invention further relates to combination therapies wherein a compound of the invention, or a pharmaceutically acceptable salt thereof, or a pharmaceutical composition or formulation comprising a compound of the invention, is administered concurrently or sequentially or as a combined preparation with another therapeutic agent or agents, for the treatment of one or more of the conditions listed.

In particular, for the treatment of the inflammatory diseases such as (but not restricted to) rheumatoid arthritis, osteoarthritis, asthma, allergic rhinitis, chronic obstructive pulmonary disease (COPD), psoriasis, and inflammatory bowel disease, the compounds of the invention may be combined with agents listed below.

Non-steroidal anti-inflammatory agents (hereinafter NSAIDs) including non-selective cyclo-oxygenase COX-1 / COX-2 inhibitors whether applied topically or systemically (such as piroxicam, diclofenac, propionic acids such as naproxen, flurbiprofen, fenoprofen, ketoprofen and ibuprofen, fenamates such as mefenamic acid, indomethacin, sulindac, azapropazone, pyrazolones such as phenylbutazone, salicylates such as aspirin); selective COX-2 inhibitors (such as meloxicam, celecoxib, rofecoxib, valdecoxib, lumarocoxib, parecoxib and etoricoxib); cyclo-oxygenase inhibiting nitric oxide donors (CINODs); glucocorticosteroids (whether administered by topical, oral, intramuscular, intravenous, or intra-articular routes); methotrexate; leflunomide; hydroxychloroquine; d-penicillamine; auranofin or other parenteral or oral gold preparations; analgesics; diacerein; intra-articular therapies such as hyaluronic acid derivatives; and nutritional supplements such as glucosamine.

The present invention still further relates to the combination of a compound of the invention, or a pharmaceutically acceptable salt thereof, together with a cytokine or agonist or antagonist of cytokine function, (including agents which act on cytokine signalling pathways such as modulators of the SOCS system) including alpha-, beta-, and gamma-interferons; insulin-like growth factor type I (IGF-1); interleukins (IL) including IL1 to 17, and interleukin antagonists or inhibitors such as anakinra; tumour necrosis factor alpha (TNF-$\alpha$) inhibitors such as anti-TNF monoclonal antibodies

(for example infliximab; adalimumab, and CDP-870) and TNF receptor antagonists including immunoglobulin molecules (such as etanercept) and low-molecular-weight agents such as pentoxyfylline.

In addition the invention relates to a combination of a compound of the invention, or a pharmaceutically acceptable salt thereof, with a monoclonal antibody targeting B-Lymphocytes (such as CD20 (rituximab), MRA-aIL16R and T-Lymphocytes, CTLA4-Ig, HuMax I1-15).

The present invention still further relates to the combination of a compound of the invention, or a pharmaceutically acceptable salt thereof, with a modulator of chemokine receptor function such as an antagonist of CCR1, CCR2, CCR2A, CCR2B, CCR3, CCR5, CCR6, CCR7, CCR8, CCR9, CCR 10 and CCR11 (for the C-C family); CXCR 1, CXCR2, CXCR3, CXCR4 and CXCR5 (for the C-X-C family) and $CX_3CR1$ for the C-$X_3$-C family.

The present invention further relates to the combination of a compound of the invention, or a pharmaceutically acceptable salt thereof, with an inhibitor of matrix metalloprotease (MMPs), i.e., the stromelysins, the collagenases, and the gelatinases, as well as aggrecanase; especially collagenase-1 (MMP-1), collagenase-2 (MMP-8), collagenase-3 (MMP-13), stromelysin-1 (MMP-3), stromelysin-2 (MMP-10), and stromelysin-3 (MMP-11) and MMP-9 and MMP-12, including agents such as doxycycline.

The present invention still further relates to the combination of a compound of the invention, or a pharmaceutically acceptable salt thereof, and a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; a N-(5-substituted)-thiophene-2-alkylsulfonamide; 2,6-di-tert-butylphenolhydrazones; a methoxytetrahydropyrans such as Zeneca ZD-2138; the compound SB-210661; a pyridinyl-substituted 2-cyanonaphthalene compound such as L-739,010; a 2-cyanoquinoline compound such as L-746,530; or an indole or quinoline compound such as MK-591, MK-886, and BAY x 1005.

The present invention further relates to the combination of a compound of the invention, or a pharmaceutically acceptable salt thereof, and a receptor antagonist for leukotrienes (LT) B4, LTC4, LTD4, and LTE4 selected from the group consisting of the phenothiazin-3-1s such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention, or a pharmaceutically acceptable salt thereof, and a phosphodiesterase (PDE) inhibitor such as a methylxanthanine including theophylline and aminophylline; a selective PDE isoenzyme inhibitor including a PDE4 inhibitor an inhibitor of the isoform PDE4D, or an inhibitor of PDE5.

The present invention further relates to the combination of a compound of the invention, or a pharmaceutically acceptable salt thereof, and a histamine type 1 receptor antagonist such as cetirizine, loratadine, desloratadine, fexofenadine, acrivastine, terfenadine, astemizole, azelastine, levocabastine, chlorpheniramine, promethazine, cyclizine, or mizolastine; applied orally, topically or parenterally.

The present invention still further relates to the combination of a compound of the invention, or a pharmaceutically acceptable salt thereof, and a proton pump inhibitor (such as omeprazole) or a gastroprotective histamine type 2 receptor antagonist.

The present invention further relates to the combination of a compound of the invention, or a pharmaceutically acceptable salt thereof, and an antagonist of the histamine type 4 receptor.

The present invention still further relates to the combination of a compound of the invention, or a pharmaceutically acceptable salt thereof, and an alpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride or ethylnorepinephrine hydrochloride.

The present invention further relates to the combination of a compound of the invention, or a pharmaceutically acceptable salt thereof, and an anticholinergic agents including muscarinic receptor (M1, M2, and M3) antagonist such as atropine, hyoscine, glycopyrrrolate, ipratropium bromide, tiotropium bromide, oxitropium bromide, pirenzepine or telenzepine.

The present invention still further relates to the combination of a compound of the invention, or a pharmaceutically acceptable salt thereof, and a beta-adrenoceptor agonist (including beta receptor subtypes 1-4) such as isoprenaline, salbutamol, formoterol, salmeterol, terbutaline, orciprenaline, bitolterol mesylate, or pirbuterol, or a chiral enantiomer thereof.

The present invention further relates to the combination of a compound of the invention, or a pharmaceutically acceptable salt thereof, and a chromone, such as sodium cromoglycate or nedocromil sodium.

The present invention still further relates to the combination of a compound of the invention, or a pharmaceutically acceptable salt thereof, with a glucocorticoid, such as flunisolide, triamcinolone acetonide, beclomethasone dipropionate, budesonide, fluticasone propionate, ciclesonide or mometasone furoate.

The present invention further relates to the combination of a compound of the invention, or a pharmaceutically

acceptable salt thereof, with an agent that modulates a nuclear hormone receptor such as PPARs.

The present invention still further relates to the combination of a compound of the invention, or a pharmaceutically acceptable salt thereof, together with an immunoglobulin (Ig) or Ig preparation or an antagonist or antibody modulating Ig function such as anti-IgE (for example omalizumab).

The present invention further relates to the combination of a compound of the invention, or a pharmaceutically acceptable salt thereof, and another systemic or topically-applied anti-inflammatory agent, such as thalidomide or a derivative thereof, a retinoid, dithranol or calcipotriol.

The present invention still further relates to the combination of a compound of the invention, or a pharmaceutically acceptable salt thereof, and combinations of aminosalicylates and sulfapyridine such as sulfasalazine, mesalazine, balsalazide, and olsalazine; and immunomodulatory agents such as the thiopurines, and corticosteroids such as budesonide.

The present invention further relates to the combination of a compound of the invention, or a pharmaceutically acceptable salt thereof, together with an antibacterial agent such as a penicillin derivative, a tetracycline, a macrolide, a beta-lactam, a fluoroquinolone, metronidazole, an inhaled aminoglycoside; an antiviral agent including acyclovir, famciclovir, valaciclovir, ganciclovir, cidofovir, amantadine, rimantadine, ribavirin, zanamavir and oseltamavir; a protease inhibitor such as indinavir, nelfinavir, ritonavir, and saquinavir; a nucleoside reverse transcriptase inhibitor such as didanosine, lamivudine, stavudine, zalcitabine or zidovudine; or a non-nucleoside reverse transcriptase inhibitor such as nevirapine or efavirenz.

The present invention still further relates to the combination of a compound of the invention, or a pharmaceutically acceptable salt thereof, and a cardiovascular agent such as a calcium channel blocker, a beta-adrenoceptor blocker, an angiotensin-converting enzyme (ACE) inhibitor, an angiotensin-2 receptor antagonist; a lipid lowering agent such as a statin or a fibrate; a modulator of blood cell morphology such as pentoxyfylline; thrombolytic, or an anticoagulant such as a platelet aggregation inhibitor.

The present invention further relates to the combination of a compound of the invention, or a pharmaceutically acceptable salt thereof, and a CNS agent such as an antidepressant (such as sertraline), an anti-Parkinsonian drug (such as deprenyl, L-dopa, ropinirole, pramipexole, a MAOB inhibitor such as selegine and rasagiline, a comP inhibitor such as tasmar, an A-2 inhibitor, a dopamine reuptake inhibitor, an NMDA antagonist, a nicotine agonist, a dopamine agonist or an inhibitor of neuronal nitric oxide synthase), or an anti-Alzheimer's drug such as donepezil, rivastigmine, tacrine, a COX-2 inhibitor, propentofylline or metrifonate.

The present invention still further relates to the combination of a compound of the invention, or a pharmaceutically acceptable salt thereof, and an agent for the treatment of acute or chronic pain, such as a centrally or peripherally-acting analgesic (for example an opioid or derivative thereof), carbamazepine, phenytoin, sodium valproate, amitryptiline or other anti-depressant agent-s, paracetamol, or a non-steroidal anti-inflammatory agent.

The present invention further relates to the combination of a compound of the invention, or a pharmaceutically acceptable salt thereof, together with a parenterally or topically-applied (including inhaled) local anaesthetic agent such as lignocaine or a derivative thereof.

A compound of the present invention, or a pharmaceutically acceptable salt thereof, can also be used in combination with an anti-osteoporosis agent including a hormonal agent such as raloxifene, or a biphosphonate such as alendronate.

The present invention still further relates to the combination of a compound of the invention, or a pharmaceutically acceptable salt thereof, together with a: (i) tryptase inhibitor; (ii) platelet activating factor (PAF) antagonist; (iii) interleukin converting enzyme (ICE) inhibitor; (iv) IMPDH inhibitor; (v) adhesion molecule inhibitors including VLA-4 antagonist; (vi) cathepsin; (vii) kinase inhibitor such as an inhibitor of tyrosine kinase (such as Btk, Itk, Jak3 or MAP, for example Gefitinib or Imatinib mesylate), a serine / threonine kinase (such as an inhibitor of a MAP kinase such as p38, JNK, protein kinase A, B or C, or IKK), or a kinase involved in cell cycle regulation (such as a cylin dependent kinase); (viii) glucose-6 phosphate dehydrogenase inhibitor; (ix) kinin-B1.- or B2. -receptor antagonist; (x) anti-gout agent, for example colchicine; (xi) xanthine oxidase inhibitor, for example allopurinol; (xii) uricosuric agent, for example probenecid, sulfinpyrazone or benzbromarone; (xiii) growth hormone secretagogue; (xiv) transforming growth factor (TGFβ); (xv) platelet-derived growth factor (PDGF); (xvi) fibroblast growth factor for example basic fibroblast growth factor (bFGF); (xvii) granulocyte macrophage colony stimulating factor (GM-CSF); (xviii) capsaicin cream; (xix) tachykinin NK1 or NK3 receptor antagonist such as NKP-608C, SB-233412 (talnetant) or D-4418; (xx) elastase inhibitor such as UT-77 or ZD-0892; (xxi) TNF-alpha converting enzyme inhibitor (TACE); (xxii) induced nitric oxide synthase (iNOS) inhibitor; (xxiii) chemoattractant receptor-homologous molecule expressed on TH2 cells, (such as a CRTH2 antagonist); (xxiv) inhibitor of P38; (xxv) agent modulating the function of Toll-like receptors (TLR), (xxvi) agent modulating the activity of purinergic receptors such as P2X7; or (xxvii) inhibitor of transcription factor activation such as NFkB, API, or STATS.

A compound of the invention, or a pharmaceutically acceptable salt thereof, can also be used in combination with an existing therapeutic agent for the treatment of cancer, for example suitable agents include:

(i) an antiproliferative/antineoplastic drug or a combination thereof, as used in medical oncology, such as an alkylating agent (for example cis-platin, carboplatin, cyclophosphamide, nitrogen mustard, melphalan, chlorambucil, busulphan or a nitrosourea); an antimetabolite (for example an antifolate such as a fluoropyrimidine like 5-fluorouracil or tegafur, raltitrexed, methotrexate, cytosine arabinoside, hydroxyurea, gemcitabine or paclitaxel); an antitumour antibiotic (for example an anthracycline such as adriamycin, bleomycin, doxorubicin, daunomycin, epirubicin, idarubicin, mitomycin-C, dactinomycin or mithramycin); an antimitotic agent (for example a vinca alkaloid such as vincristine, vinblastine, vindesine or vinorelbine, or a taxoid such as taxol or taxotere); or a topoisomerase inhibitor (for example an epipodophyllotoxin such as etoposide, teniposide, amsacrine, topotecan or a camptothecin);

(ii) a cytostatic agent such as an antioestrogen (for example tamoxifen, toremifene, raloxifene, droloxifene or iodoxyfene), an oestrogen receptor down regulator (for example fulvestrant), an antiandrogen (for example bicalutamide, flutamide, nilutamide or cyproterone acetate), a LHRH antagonist or LHRH agonist (for example goserelin, leuprorelin or buserelin), a progestogen (for example megestrol acetate), an aromatase inhibitor (for example as anastrozole, letrozole, vorazole or exemestane) or an inhibitor of 5α-reductase such as finasteride;

(iii) an agent which inhibits cancer cell invasion (for example a metalloproteinase inhibitor like marimastat or an inhibitor of urokinase plasminogen activator receptor function);

(iv) an inhibitor of growth factor function, for example: a growth factor antibody (for example the anti-erbb2 antibody trastuzumab, or the anti-erbb1 antibody cetuximab [C225]), a farnesyl transferase inhibitor, a tyrosine kinase inhibitor or a serine/threonine kinase inhibitor, an inhibitor of the epidermal growth factor family (for example an EGFR family tyrosine kinase inhibitor such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) or 6-acrylamido-N-(3-chloro-4-fluorophenyl)-7-(3-morpholinopropoxy)quinazolin-4-amine (CI 1033)), an inhibitor of the platelet-derived growth factor family, or an inhibitor of the hepatocyte growth factor family;

(v) an antiangiogenic agent such as one which inhibits the effects of vascular endothelial growth factor (for example the anti-vascular endothelial cell growth factor antibody bevacizumab, a compound disclosed in WO 97/22596, WO 97/30035, WO 97/32856 or WO 98/13354), or a compound that works by another mechanism (for example linomide, an inhibitor of integrin αvβ3 function or an angiostatin);

(vi) a vascular damaging agent such as combretastatin A4, or a compound disclosed in WO 99/02166, WO 00/40529, WO 00/41669, WO 01/92224. WO 02/04434 or WO 02/08213;

(vii) an agent used in antisense therapy, for example one directed to one of the targets listed above, such as ISIS 2503, an anti-ras antisense;

(viii) an agent used in a gene therapy approach, for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; or (ix) an agent used in an immunotherapeutic approach, for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The present invention will now be further explained by reference to the following illustrative examples. In the examples the NMR spectra were measured on a Varian Unity spectrometer at a proton frequency of either 300 or 400 MHz. The MS spectra were measured on either an Agilent 1100 MSD G1946D spectrometer or a Hewlett Packard HP1100 MSD G1946A spectrometer. Preparative HPLC separations were performed using a Waters Symmetry® or Xterra® column using 0.1% aqueous trifluoroacetic acid: acetonitrile, 0.1% aqueous ammonia: acetonitrile or 0.1% ammonium acetate: acetonitrile as the eluant.

**Example 1**

**2-Chloro-3-fluoro-N-(5-fluoro-3-methoxypyrazin-2-yl)- benzenesulfonamide**

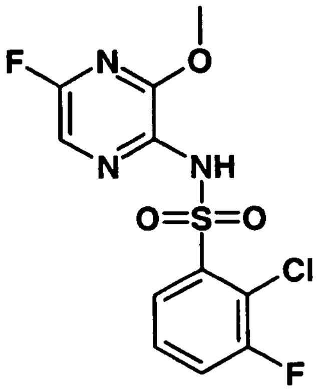

a) **3,5-Dibromo-2.(2,5.dimethyl-1*H*-pyrrol-1-yl)-pyrazine**

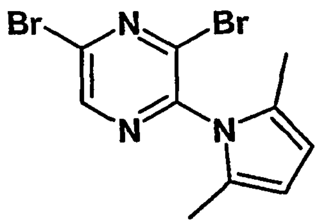

3,5-Dibromo-2-pyrazinamine (Synthesis, 1990, p659-660) (6.33g), acetonylacetone (4.42g) and p-toluenesulphonic acid (0.4g) in toluene (100ml) was heated under reflux using a Dean and Stark trap. After 2h, the reaction mixture was allowed to cool, evaporated under reduced pressure to approximately 15ml. The solution was diluted with dichloromethane and passed through a silica gel column eluting with dichloromethane. After evaporation of the solvent, the product crystallised on standing. Yield 8.00g.
m/e 330/332/334 (M+1)

b) **2-(2,5-dimethyl-1*H*-pyrrol-1-yl)-3,5-difluoro-pyrazine**

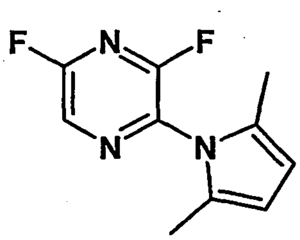

3,5-Dibromo-2-(2,5-dimethyl-1*H*-pyrrol-1-yl)-pyrazine (product from step a) (7.3g), anhydrous potassium fluoride (4.2g) and 18-crown-6 (0.2g) in anhydrous 2-methoxyethyl ether (30ml), under nitrogen were heated at 120°C for 16h. After cooling, the mixture was partitioned between water and dichloromethane. The dichloromethane solution was washed with water and then passed through a large pad of silica gel eluting with dichloromethane. The solvent was evaporated to afford the product. Yield 4.5g.
1H NMR (D6-DMSO) δ 8.37 (1H, dd), 5.96 (2H, s), 2.07 (6H, s).

**c) 3,5-Difluoro-2-pyrazinamine**

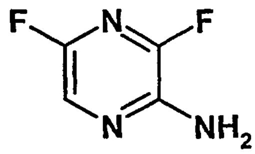

2-(2,5-Dimethyl-1*H*-pyrrol-1-yl)-3,5-difluoro-pyrazine (product of step b) (0.4g) in water (6ml) and HCl in dioxane (20ml of a 4M solution) was heated at 50°C for 16h. The solution was then concentrated to about 8ml and partitioned between water and ethyl acetate. The ethyl acetate layer was dried ($MgSO_4$) and evaporated. Purification was by silica gel chromatography eluting with ethyl acetate:iso-hexanes 1:3. The solvent was evaporated to afford the product. Yield 0.09g.
1H NMR ($CDCl_3$) δ 7.77 (1H, dd), 4.70 (2H, br s).

**d) 5-Fluoro-3-methoxy-2-pyrazinamine**

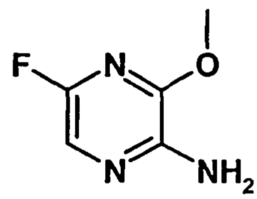

3,5-Difluoro-2-pyrazinamine (product of step c) (0.09g) and sodium methoxide (0.3ml of a 25% solution in methanol) in methanol (2ml) were stirred at room temperature. After 0.5h, the solution was partitioned between ethyl acetate and saturated aqueous ammonium chloride. The ethyl acetate layer was dried ($MgSO_4$) and evaporated to give the product. Yield 0.06g.
1H NMR ($CDCl_3$) δ 7.37 (1H, d), 4.65 (2H, br s), 4.00 (3H, s).

**e) 3-Fluoro-*N*-(5-fluoro-3-methoxypyrazin-2-yl)-benzenesulfonamide**

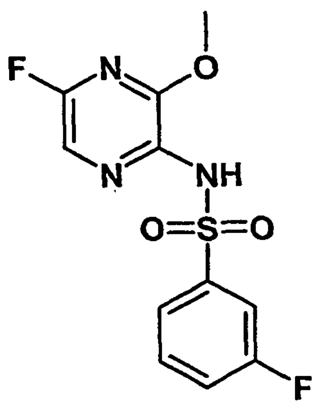

Potassium tert-butoxide (4ml of 1M solution in tetrahydrofuran) was added dropwise to a stirred solution of 5-fluoro-3-metboxy-2-pyrazinamine (product of step d) (0.25g) and 3-fluorobenzenesulfonyl chloride (0.43g) in dry tetrahydrofuran (5ml) cooled in an ice bath. After 0.5h, the reaction mixture was quenched with 2M aqueous hydrochloric acid (50ml). The mixture was extracted with ethyl acetate. The ethyl acetate layer was dried ($MgSO_4$ and evaporated. Purification was by silica gel chromatography eluting with ethyl acetate:iso-hexanes 1:3. The solvent was evaporated to afford the product. Yield 0.42g.
1HNMR (D6-DMSO) δ 11.27 (1H, br s), 7.73-7.90 (3H, m), 7.67-7.73 (1H, m), 7.50-7.60 (1H, m), 3.92 (3H, s).

**f) 2-Chloro-3-fluoro-*N*-(5-fluoro-3-methoxypyrazin-2-yl)-benzenesulfonamide**

3-Fluoro-*N*-(5-fluoro-3-methoxypyrazin-2-yl)-benzenesulfonamide (product from step e)(0.19g) in dry tetrahydrofuran (3ml) was added dropwise to a stirred solution of LDA (made by adding 0.56ml of 2.SM BuLi in hexanes to diisopropylamine (0.18g)) in dry tetrahydrofuran (7ml) at -78°C. After 15 minutes, hexachloroethane (0.6g) in dry tetrahydrofuran (3ml) was added dropwise. After 1h, the cooling bath was removed and the solution allowed to warm to room temperature. The reaction mixture was partitioned between ethyl acetate and 2M aqueous hydrochloric acid. The organic layer was evaporated. Purification was by silica gel chromatography eluting with ethyl acetate:iso-hexanes 1:3. The solvent was evaporated to afford the product. Yield 0.13g.
m/e 336/338 (M+1)
1H NMR (D6-DMSO) δ 11.54 (1H, br s), 7.91 (1H, dd), 7.7-7.8 (2H, m), 7.61 (1H, dt), 3.90 (3H, s).
13C NMR (D6-DMSO) δ 157.9 (d, J 249 Hz), 154.7 (d, J 248 Hz), 149.4 (d, J 8.8 Hz), 140.4, 134.1 (d, J 3.6 Hz), 128.7 (d, J 8.2 Hz), 126.7 (d, J 3.2 Hz), 121.0 (d, J 21.8 Hz), 118.1 (d, J 38.3), 117.8 (d, 20.4 Hz), 54.7.

**Example 2**

**2,3-dichloro-*N*-(5-fluoro-3-methoxypyrazin-2-yl)-benzenesulfonamide**

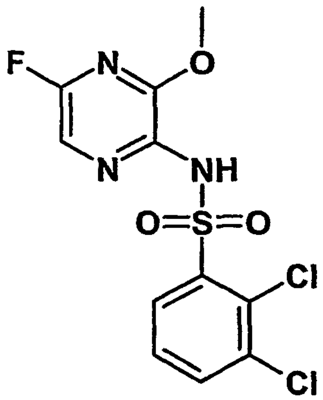

**a) 2,3-dichloro-*N*-(3-methoxy-5-nitropyrazin-2-yl)-benzenesulfonamide**

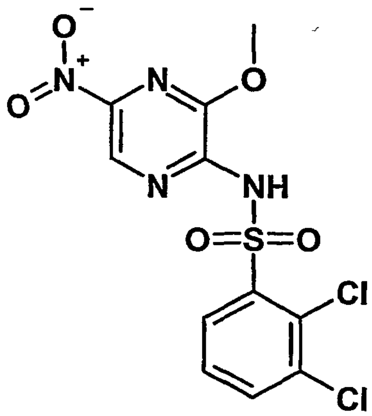

Fuming nitric acid (1.26g) was added dropwise to a stirred suspension of 2,3-dichloro-*N*-(3-methoxypyrazin-2-yl)-benzenesulfonamide (WO2003059893, example 30) (4.5g) in acetic acid (45ml) at room temperature. The reaction was carefully heated to 75°C. After 1h, the reaction mixture was allowed to cool and the white crytalline product collected by filtration. Yield 3.94g.
1H NMR (D6-DMSO) δ 8.53 (1H, s), 8.16 (1H, d), 7.95 (1H, d), 7.61 (1H, t) 4.02 (3H, s).

**b) *N*-(5-amino-3-methoxypyrazin-2-yl)-2,3-dichloro-benzenesulfonamide**

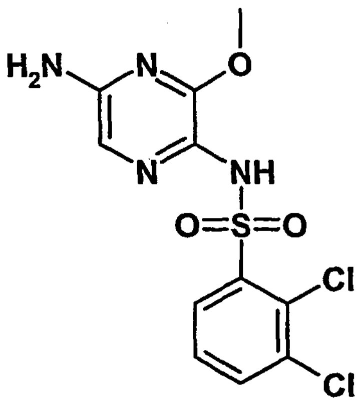

2,3-Dichloro-*N*-(3-methoxy-5-nitropyrazin-2-yl)-benzenesulfonamide (product of step 2a) (4g) and 5% palladium on charcoal (Johnson Matthey type 440 paste) (0.8g) in acetic acid (40ml) was heated at 60°C under a hydrogen atmosphere (1 bar) until hydrogen uptake ceased (16h). After cooling to room temperature the precipitated product and palladium catalyst was collected by filtration and washed with a little acetic acid. The solid was suspended in tetrahydrofuran (500ml) and stirred for 1h. The palladium catalyst was removed by filtration through celite. The tetrahydrofuran solution was evaporated to dryness and toluene added to the solid and evaporated under reduced pressure to give a light brown solid. Yield 2.6g
1H NMR (D6-DMSO) δ 10.04 (1H, s), 7.91-7.88 (2H, m), 7.50 (1H, t), 7.08 (1H, s), 6.43 (2H, br s), 3.59 (3H, s).

**c) 2,3-dichloro-*N*-(5-fluoro-3-methoxypyrazin-2-yl)-benzenesulfonamide**

Sodium nitrite (0.44g) was added portionwise to a stirred solution of *N*-(5-amino-3-methoxypyrazin-2-yl)-2,3-dichloro-benzenesulfonamide (product of step 2b) (2g) in acetontrile (10ml) and 48% aqueous $HBF_4$ (25ml) cooled in an ice bath. After 1h, the reaction mixture was poured on to water (250ml) and extracted with ethyl acetate. The ethyl acetate solution was evaporated to dryness and the product purified by silica gel chromatography eluting with ethyl acetate:iso-hexanes 1:4. The solvent was evaporated to afford the product. Yield 0.4g.
m/e 350/352/354 (M-1)

1H NMR (D6-DMSO) δ 8.05 (1H, dd), 7.95 (1H, dd), 7.73 (1H, d), 7.59 (1H, t), 3.90 (3H, s)
13C NMR (D6-DMSO) δ 154.2 (d, J 256 Hz), 149.0 (d, J 7.9 Hz), 140.3, 134.1, 133.6, 133.2, 129.4, 128.1, 127.9, 117.5 (d, J 37.5 Hz), 54.2

**Example 3**

**2,3-Dichloro-*N*-(6-fluoro-3-methoxypyrazin-2-yl)-benzenesulfonamide**

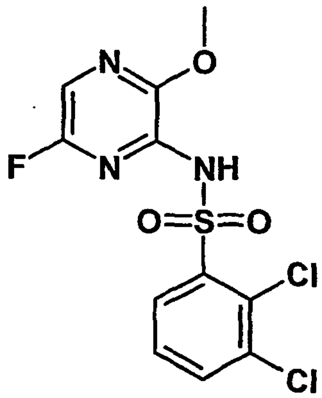

**a) *N*-(5-Bromo-3-methoxy-6-nitropyrazin-2-yl)-2,3-dichloro-benzenesulfonamide**

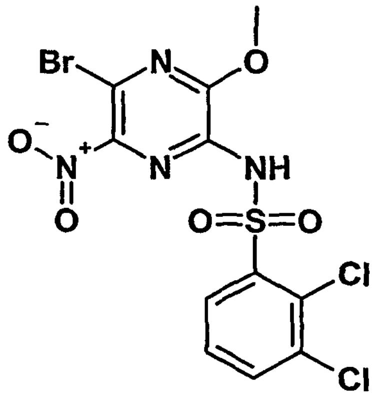

Nitronium tetrafluoroborate (7.5g) was added portionwise over about 15 minutes to a stirred suspension of *N*-(5-bromo-3-methoxypyrazin-2-yl)-2,3-dichloro-benzenesulfonamide (WO2003059893, example 8) (10.0g) in acetonitrile (100ml). After 2h, further nitronium tetrafluoroborate (0.75g) was added. After a further 1h, the reaction mixture was poured on to ice/water and extracted with dichloromethane. The extracts were dried ($MgSO_4$) and evaporated. Purification was by silica gel chromatography eluting with ethyl acetate:iso-hexanes 1:1. The solvent was evaporated to afford the product. Yield 8.4g.
1H NMR ($CDCl_3$) δ 8.36 (1H, m), 7.74 (1H, m), 7.49 (1H, t), 4.18 (3H, s).

**b) *N*-(6-Amino-5-bromo-3-methoxypyrazin-2-yl)-2,3-dichloro-benzenesulfonamide**

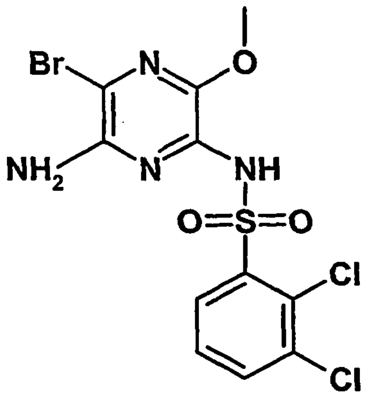

*N*-(5-Bromo-3-methoxy-6-nitropyrazin-2-yl)-2,3-dichloro-benzenesulfonamide (product of step 3a) (7.4g) in ethyl acetate (100ml) and acetic acid (50ml) containing 5% palladium on charcoal (Johnson Matthey type 39 paste) (3.2g) was put under hydrogen (1 bar) with vigorous stirring. After 3h, the reaction mixture was filtered through a pad of celite and evaporated. Yield 6.5g.
m/e 427/429 (M+1)

**c) *N*-(5-Bromo-6-fluoro-3-methoxypyrazin-2-yl)-2,3-dichloro-benzenesulfonamide**

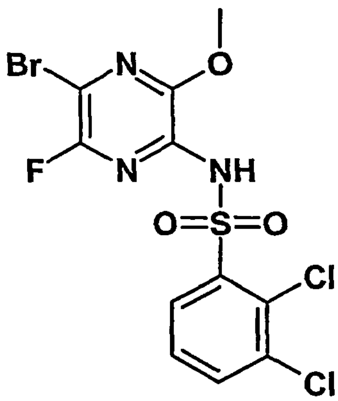

Sodium nitrite (2.4g) was added portionwise over about 20 minutes to a stirred solution of *N*-(6-Amino-5-bromo-3-methoxypyrazin-2-yl)-2,3-dichloro-benzenesulfonamide (product of step 3b) (7.4g) in hydrogen fluoride-pyridine (pyridinium poly(hydrogen fluoride)) (30ml) cooled to -10°C. After 0.5h, water was added and the solution extracted with dichloromethane (x2). The combined extracts were washed with water and then passed through a silica gel pad eluting with 1.25% methanol in dichloromethane. Purification was by silica gel chromatography eluting with methanol: dichloromethane 1:100. The solvent was evaporated to afford the product. Yield 4.1g.
m/e 430/432 (M+1)

**d) 2,3-Dichloro-*N*-(6-fluoro-3-methoxypyrazin-2-yl)-benzenesulfonamide**

*N*-(5- Bromo-6-fluoro-3-methoxypyrazin-2-yl)-2,3-dichloro-benzenesulfonamide (product from step 3c) (0.2 g) in ethyl acetate (10ml) and triethylamine (1ml) containing 5% palladium on charcoal (Johnson Matthey type 39 paste) (0.4g) was put under hydrogen (1 bar) with vigorous stirring. After 0.5h, the reaction mixture was filtered through a pad of celite and evaporated. Purification was by silica gel chromatography eluting with ethyl acetate:iso-hexanes 1:4. The solvent was evaporated to afford the product. Yield 0.06g.
m/e 352/354/356 (M+1)
1H NMR (D6-DMSO) δ 8.13 (1H, dd), 7.95 (1H, dd), 7.75 (1H, d), 7.62 (1H, t), 3.91 (3H, s)

13C NMR (D6-DMSO) δ 152.7 (d, J 240.4 Hz), 147.7 (d, J 147.7 Hz), 140.0, 134.9, 134.1 (d, J 10.3Hz), 133.8, 130.6, 128.8, 128.4, 118.9 (d 39 Hz), 54.5.

**Example 4**

**2,3-Dichloro-*N*-[6-fluoro-5-(hydroxymethyl)-3-methoxypyrazin-2-yl]-benzenesulfonamide**

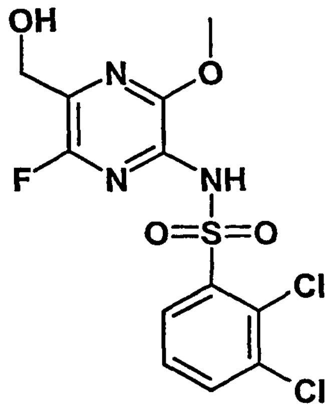

**a) Methyl 5-{[(2,3-dichlorophenyl)sulfonyl]amino}-3-fluoro-6-methoxypyrazine-2-carboxylate**

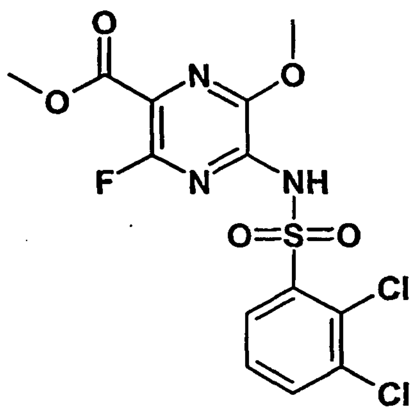

*N*-(5-Bromo-6-fluoro-3-methoxypyraz-2-inyl)-2,3-dichloro-benzenesulfonamide (product of example 3c) (0.4g) and dichloro[1,1'-bis(diphenylphosphino)ferrocene] palladium(II) dichloromethane adduct (0.06g) in methanol (15ml) and triethylamine (5ml) was heated at 90-100°C under an atmosphere of carbon monoxide (6 bar). After 3h, the reaction was allowed to cool and the solution evaporated. The residue was partitioned between ethyl acetate and aqueous 2M hydrochloric acid. The aqueous layer was extracted with ethyl acetate and the combined extracts dried ($MgSO_4$) and evaporated. Purification was by silica gel chromatography eluting with ethyl acetate. The solvent was evaporated to afford the product. Yield 0.25g.
m/e 410/412 (M+1)

**b) 2,3 Dichloro-*N*-[6-fluoro-5-(hydroxymethyl)-3-methoxypyrazin-2-yl]-benzenesulfonamide**

Lithium triethylborohydride (Superhydride, 2ml of 1M solution in tetrahydrofuran) was added over 1 minute to a stirred solution of methyl 5-{[(2,3-dichlorophenyl)sulfonyl] amino}-3-fluoro-6-methoxypyrazine-2-carboxylate(product

of step 4a) (0.17g) in dry tetrahydrofuran (5ml) cooled in an ice bath. After 20 minutes, the reaction mixture was partitioned between ethyl acetate and saturated aqueous citric acid. The combined ethyl acetate extract was washed with water, dried ($MgSO_4$) and evaporated. Purification was by silica gel chromatography eluting with ethyl acetate:iso-hexanes 1: 1. The solvent was evaporated to afford the product. Yield 0.035g.
m/e 382/384/386 (M+1)
1H NMR (D6-DMSO) δ 8.11 (1H, dd), 7.95 (1H, dd), 7.61 (1H, t), 4.37 (2H, s) 3.91 (3H, s)
13C NMR (D6-DMSO) δ150.1 (d, J 241.1 Hz), 147.1 (d, J 2 Hz), 140.0, 134.9, 133.8, 132.8 (d, J 10.1 Hz), 130.9 (d, J 30.4 Hz), 130.6, 128.7, 128.4, 58.2, 54.5

**Example 5**

**3-Chloro-2-fluoro-*N*-(5-fluoro-3-methoxypyrazin-2-yl)-benzenesulfonamide**

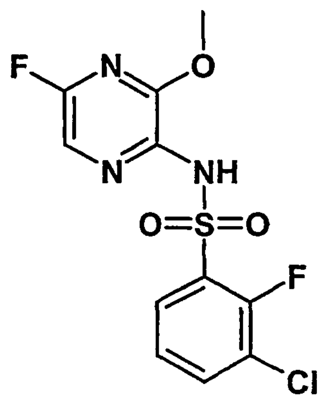

**a) 3-Chloro-2-fluoro-*N*-(3-methoxypyrazin-2-yl)-benzenesulfonamide**

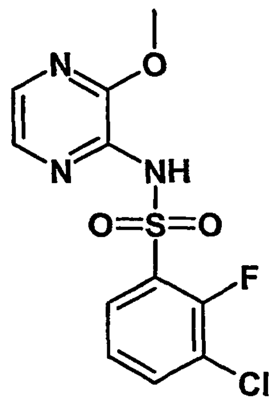

Prepared by the method of example 1 step e using 3-chloro-2-fluorobenzenesulphonyl chloride (2.5g) and 3-methoxy-2-pyrazinamine (1.25g). After workup the combined ethyl acetate extracts were dried ($MgSO_4$ and evaporated to give a light brown solid. Yield 3.3g.
m/e 318/320 (M+1)

**b) 3-Chloro-2-fluoro-*N*-(3-methoxy-5-nitropyrazin-2-yl)-benzenesulfonamide**

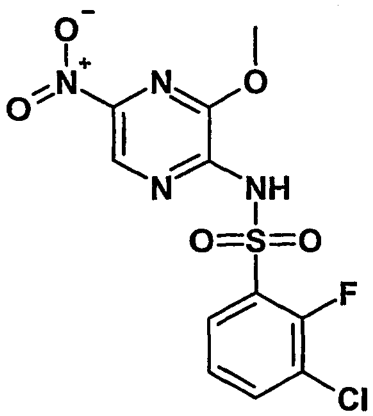

3-Chloro-2-fluoro-*N*-(3-methoxypyrazin-2-yl)-benzenesulfonamide (product of step 5a) (2.5g) was added to nitronium tetrafluoroborate in sulfolane (50ml of 0.5M solution) and the mixture heated at 50°C. After 6h, further nitronium tetrafluoroborate in sulfolane (20ml) was added. After a further 3h, the mixture was cooled and poured onto ice/water. The resulting oil was dissolved in ethyl acetate and separated. The combined ethyl acetate extracts were dried ($MgSO_4$) and evaporated to dryness to give an orange oil. Purification was by silica gel chromatography eluting with dichloromethane to remove the sulfolane then ethyl acetate to collect the product. The product was dissolved in dichloromethane and washed with water to remove the residual sulfolane. The organic solution was dried ($MgSO_4$ and evaporated. Yield 1.2g.
1H NMR (D6-DMSO) δ 8.53 (1H, s), 7.95 (1H, t), 7.88 (1H, t), 7.42 (1H, t), 3.99 (3H, s)

**c) *N*-(5-Amino-3-methoxypyrazin-2-yl)-3-chloro-2-fluoro-benzenesulfonamide**

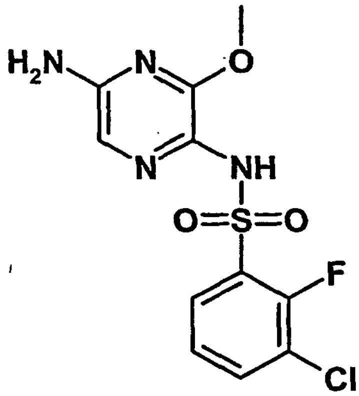

3-Chloro-2-fluoro-*N*-(3-methoxy-5-nitropyrazin-2-yl)-benzenesulfonamide (product of step 5b) (0.8g), iron powder (0.8g) and ammonium chloride (0.8g) in ethanol (40ml) and water (40ml) was heated under reflux. After 1h the reaction was allowed to cool and filtered through celite, washing well with methanol. The solution was evaporated to dryness then partitioned between ethyl acetate and water. The organic layer was dried ($MgSO_4$) and evaporated. Purification was by silica gel chromatography eluting with ethyl acetate. The solvent was evaporated to afford the product. Yield 0.28g.
1H NMR (D6-DMSO) δ 7.87 (1H, t), 7.67 (1H, t), 7.35 (1H, t), 7.11 (1H, s), 6.47 (2H, s), 3.58 (3H, s)

**d) 3-Chloro-2-fluoro-*N*-(5-fluoro-3-methoxypyrazin-2-yl)-benzenesulfonamide**

The title compound was prepared using the method of example 2 step c using *N*-(5-amino-3-methoxypyrazin-2-yl)-3-chloro-2-fluoro-benzenesulfonamide (product of step 5c) (0.27g). Purification was by silica gel chromatography eluting with ethyl acetate:iso-hexanes 1:3. The solvent was evaporated to afford the product. Yield 0.11g.

m/e 336/338 (M+1)
1H NMR (D6-DMSO) δ 7.92 (1H, t), 7.86 (1H, t), 7.76 (1H, d), 7.43 (1H, t), 3.90 (3H, s)
13C NMR (D6-DMSO) δ 154.8 (d, J 249 Hz), 153.5 (d, J 258 Hz), 149.7 (d, J 9.1 Hz), 135.4, 133.9 (d, J 3.9 Hz), 130.7 (d, 13.4 Hz), 129.1, 125.5 (d, J 5.0 Hz), 121.1 (d, J 17.2 Hz), 118.1 (d, J 38.3 Hz), 54.7

**Example 6**

**2,3-Dichloro-*N*-[5-fluoro-6-(hydroxymethyl)-3-methoxypyrazin-2-yl]-**benzenesulfonamide

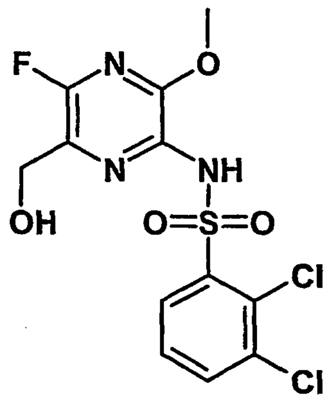

**a) 2,3-Dichloro-*N*-(5-fluoro-3-methoxy-6-nitropyrazin-2-yl)-benzenesulfonamide**

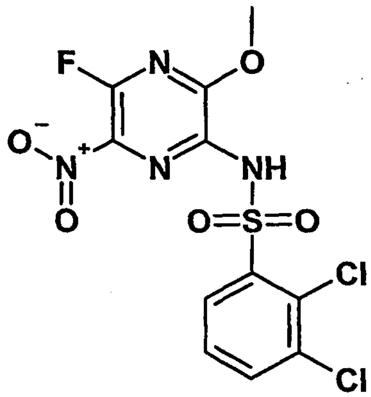

Nitronium tetrafluoroborate (0.4g) was added portionwise to a stirred suspension of 2,3-dichloro-*N*-(5-fluoro-3-methoxypyrazin-2-yl)-benzenesulfonamide (example 2) (0.5g) in acetonitrile (10ml). After 2h, the reaction was partitioned between ethyl acetate and water. The ethyl acetate extract was dried ($MgSO_4$) and evaporated. Purification was by silica gel chromatography eluting with ethyl acetate:iso-hexanes 1:1. The solvent was evaporated to afford the product. Yield 0.3g.
1H NMR (D6-DMSO) δ 8.25 (1H, dd), 7.94 (1H, dd), 7.61 (1H, t), 4.03 (3H, s)

**b) *N*-(6-Amino-5-fluoro-3-methoxypyrazin-2-yl)-2,3-dichloro-benzenesulfonamide**

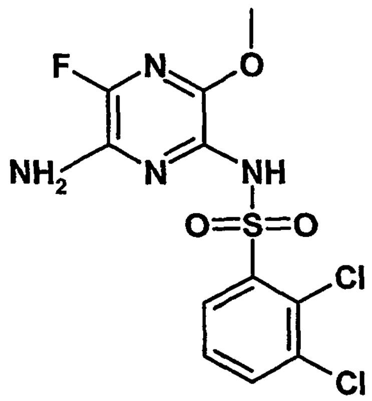

2,3-Dichloro-*N*-(5-fluoro-3-methoxy-6-nitropyrazin-2-yl)-benzenesulfonamide (product of step 6a) (0.95g) in ethyl acetate (10ml) and acetic acid (5ml) containing 5% palladium on charcoal (Johnson Matthey type 39 paste) (0.3g) was put under hydrogen (1 bar) with vigorous stirring. After 16h, the reaction mixture was filtered through a pad of celite and evaporated. Yield 0.75g.
m/s 367/369/371 (M+1)

**c) *N*-(6-Bromo-5-fluoro-3-methoxypyrazin-2-yl)-2,3-dichloro-benzenesulfonamide**

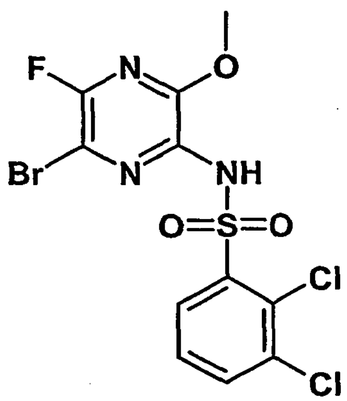

Sodium nitrite (0.2g) was added portionwise to a stirred solution of *N*-(6-Amino-5-fluoro-3-methoxypyrazin-2-yl)-2,3-dichloro-benzenesulfonamide (product from step 6b) (0.5g) in acetonitrile (5ml) and 48% aqueous HBr (5ml) cooled to -10°C. After 20 min, the reaction mixture was partitioned between dichloromethane and water. The organic extract was dried ($MgSO_4$) and evaporated. Purification was by silica gel chromatography eluting with dichloromethane. The solvent was evaporated to afford the product. Yield 0.15g.
m/s 430/432 (M+1)

**d) Methyl 6-{[(2,3-dichlorophenyl)sulfonyl]amino}-3-fluoro-5-methoxypyrazine-2-carboxylate**

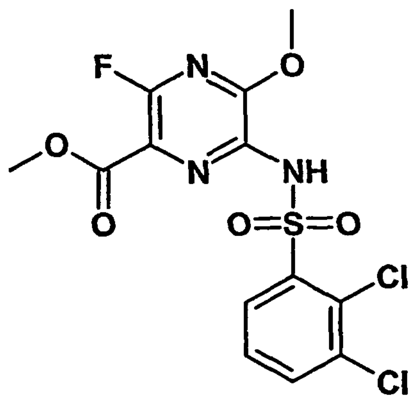

*N*-(6-Bromo-5-fluoro-3-methoxypyrazin-2-yl)-2,3-dichloro-benzenesulfonamide (product of example 6c) (0.14g) and dichloro[1,1'-bis(diphenylphosphino)ferrocene] palladium(II) dichloromethane adduct (0.2g) in methanol (10ml) and triethylamine (5ml) was heated at 90-100°C under an atmosphere of carbon monoxide (6 bar). After 16h, the reaction was allowed to cool and the solution evaporated. The residue was partitioned between ethyl acetate and aqueous 2M hydrochloric acid. The aqueous layer was extracted with ethyl acetate and the combined extracts dried ($MgSO_4$) and evaporated. Purification was by silica gel chromatography eluting with ethyl acetate:iso-hexanes 1:2. The solvent was evaporated to afford the product. Yield 0.1 g.
m/e 410/412 (M+1)

**e) 2,3-Dichloro-*N*-[5-fluoro-6-(hydroxymethyl)-3-methoxypyrazinyl]-benzenesulfonamide**

Lithium triethylborohydride (Superhydride, 1.5ml of 1M solution in tetrahydrofuran) was added over 1 minute to a stirred solution of methyl 6-{[(2,3-dichlorophenyl)sulfonyl] amino}-3-fluoro-5-methoxypyrazine-2-carboxylate (product of step 6d) (0.1g) in dry tetrahydrofuran (2ml) cooled in an ice bath. After 20 minutes, the reaction mixture was partitioned between ethyl acetate and aqueous 2M HCl. The combined ethyl acetate extract was washed with water, dried ($MgSO_4$) and evaporated. Purification was by silica gel chromatography eluting with ethyl acetate:iso-hexanes 2: 1. The solvent was evaporated to afford the product. Yield 0.02g.
m/e 382/384/386 (M+1)
1H NMR (D6-DMSO) δ 11.49 (1H, br s), 8.10 (1H, dd), 7.94 (1H, dd), 7.59 (1H, t), 4.25 (2H, s), 3.85 (3H, s)

**Example 7**

**3-Chloro-*N*-(5-fluoro-3-methoxypyrazin-2-yl)-2-methyl-benzenesulfonamide**

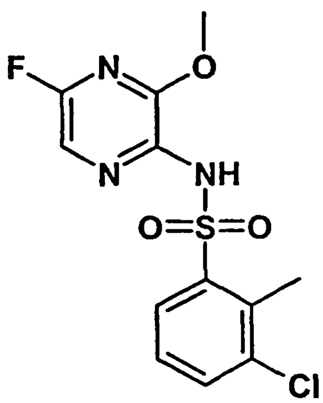

The title compound was prepared from 3-chloro-2-methylbenzenesulfonyl chloride (0.18g) and 5-fluoro-3-methoxy-2-pyrazinamine (product of example 2 step d) (0.1g) using the method of example 1 step e. Purification was by silica

gel chromatography eluting with ethyl acetate: iso-hexanes 1:4. The solvent was evaporated to afford the product. Yield . 0.15g.
H NMR (D6-DMSO) δ 11.34 (1H, br s), 7.95 (1H, d), 7.73 (1H, d), 7.70 (1H, d), 7.43 (1H, t), 3.92 (3H, s), 2.66 (3H, s)

**Pharmacological Data**

**FMAT Whole cell binding assay**

*Cells*

CHO-K1 cells stably expressing the human recombinant CCR4 receptor (Euroscreen; Brussels, Belgium) were cultured in NUT.MIX.F_12(HAM) medium with glutamax-1, containing 10% (v/v) foetal bovine serum and 400 μg $ml^{-1}$ geneticin.
Cells were harvested at approximately 70% confluence by treatment with a cell dissociation buffer, and seeded at $5x10^3$ cells/100μl culture medium into wells of a black Costar clear-bottomed 96-well microtitre plates. Plates were incubated overnight at 37°C in 5% $CO_2$ and used the following day.

*Assay*

Before use, the cell plates were washed twice with 100 μl Hanks balanced salt solution (HBSS). To each well was then added 65 μl of HBSS, 10 μL of 10% DMSO in HBSS ± test compound and then 25 μl of 2.8 nM FB-MDC (Applied Biosystems). This fluorescent probe was prepared from a 10 μM stock in 0.08% (v/v) TFA/16% (v/v) acetonitrile, diluted into HBSS.
After two hours incubation in the dark at room temperature, the plates were analysed in an FMAT8100 reader (Applied Biosystems) to measure fluorescence that was associated with binding of FB-MDC to the cells. Compound activity was determined as an $pIC_{50}$ [-log(concentration of compound that results in 50% inhibition)], comparing fluorescence in control and background wells.

**Measurement of Plasma Protein Binding**

The extent of plasma protein binding was determined via equilibrium dialysis of a compound between human plasma and aqueous buffer at 37°C and determination of the concentration of compound in the plasma and buffer by HPLC-MS/MS.

*Method*

Dialysis cells (molecular weight cut-off 5000) were prepared by rinsing with water followed by soaking in the dialysis buffer for a minimum of 1 hour. The dialysis buffer was isotonic buffered saline pH 7.4. Stock solutions of compound in dimethylsulphoxide were prepared at a concentration of 1mM. Frozen pooled Human plasma was obtained from volunteers.
The stock DMSO solution of a compound was added to the plasma at a ratio of 10 μl of DMSO to each ml of plasma. This gave a 1% DMSO in plasma solution with each compound at a concentration of 10 μM.
Dialysis cells were then prepared and one half of the cell filled with 750 μl of dialysis buffer and the other half of the cell with 750 μl of plasma solution of compound. Once prepared the cells were sealed and immersed in a water bath at 37°C. These cells were then rotated for a minimum of 4 hours to equilibrate.
After equilibration 500 μl of the buffer samples were removed and added to HPLC vials along with 100 μl of plasma (sample in 6-fold diluted plasma), and 100 μl of the plasma samples were removed and added to HPLC vials along with 500 μl of dialysis buffer (sample in 6-fold diluted plasma).
The samples were then analysed using HPLC-MS/MS. A four point calibration curve was obtained by dilutions of the stock solutions with 6-fold diluted plasma at concentrations of 0.05 μM, 0.15 μM, 0.5 μM and 2.5 μM which were injected in this order followed by the buffer sample and then the plasma sample.

*Calculation*

The concentration of compound in the samples were determined using MassLynx version 4.0 software (produced by Waters/Micromass) that automatically calculated a calibration curve and the concentration of compound in the cells. Plasma protein binding was determined from the calibration curve as the percentage of compound bound in human plasma (% bound) using the following equation wherein the factor in the numerator accounts for the small dilution of the

aqueous samples with plasma and the factor of 6 in the denominator serves to correct for the 6-fold dilution of the plasma samples with buffer;

$$\%\text{bound} = 100 - 100\left(\frac{1.2\left(\dfrac{\text{Buffer concentration x Standard Injection vol.}}{\text{Buffer injection vol.}}\right)}{6\left(\dfrac{\text{Plasma concentration x Standard injection vol.}}{\text{Plasma injection vol.}}\right)}\right).$$

**Whole Blood Potency**

Predicted whole blood potency is a measure of the combined effects of CCR4 activity and plasma protein binding, and is calculated by the formula: Whole Blood Potency = CCR4 pIC50 + Log ((100- %Bound)/100).

**Results**

**Table 1** shows the CCR4 $pIC_{50}$ , plasma protein binding (%bound) figures and predicted whole blood potency for Examples 1-7 according to the present invention and comparative compounds from WO03/059893. The comparative compounds are the analogous chlorine-containing and bromine-containing compounds exemplified in WO03/059893 (Example 5, 2,3-dichloro-N-(5-chloro-3-methoxy-2-pyrazinyl)benzenesulphonamide; and Example 8, 2,3-dichloro-N-(5-bromo-3-methoxy-2-pyrazinyl)benzenesulphonamide) and Example 30 (2,3-dichloro-N-(3-methoxy-2-pyrazinyl)benzenesulphonamide).

**Table 1**

| Compound of Example No. | FMAT CCR4 $pIC_{50}$ | % Bound Human Plasma | Whole Blood Potency |
|---|---|---|---|
| 1 | 8.3 | 99.0 | 6.3 |
| 2 | 8.5 | 99.4 | 6.3 |
| 3 | 8.5 | 99.7 | 6.0 |
| 4 | 7.8 | 98.7 | 5.9 |
| 5 | 8.0 | 98.9 | 6.0 |
| 6 | 8.2 | 99.5 | 5.9 |
| 7 | 8.4 | 99.6 | 6.0 |
| Ex. 5 WO03/059893 | 7.9 | 99.8 | 5.2 |
| Ex. 8 WO03/059893 | 8.3 | 99.8 | 5.6 |
| Ex. 30 WO03/059893 | 7.7 | 98.9 | 5.7 |

The whole blood potency of the compounds of the present invention, wherein the pyrazine ring is substituted with fluorine in the 5 or 6 positions, is significantly higher than for the comparative compounds wherein the pyrazine is subsitituted with chlorine or bromine. The combination of very high potency and low plasma protein binding to human plasma makes the fluorine-containing compounds of the present invention more efficacious in vivo.

**Metabolite Testing**

5-Fluoro-3-methoxy-2-pyrazinamine and 6-fluoro-3-methoxy-2-pyrazinamine were tested for mutagenicity according to the test procedure of Maron and Ames described in Mutation Res. 1983; 113:173-215 using *salmonella typhimurium* LT2 strains TA98 and TA100. For metabolic activation a homogenate of liver from Aroclor 1254-treated

rats (post-mitochondrial fraction (S9) purchased from Molecular Toxicology Inc., Boone, North Carolina, USA) was added to agar plates (without histidine) together with the test compound and the bacterial tester strains; the complete activation system employed was: phosphate buffer (0.1 mol/L, pH 7.4):100 mmol/L; magnesium chloride: 8 mmol/L; potassium chloride: 33 mmol/L; nicotinamide adenine dinucleotide phosphate: 4 mmol/L; glucose-6-phosphate:5 mmol/L; and rat liver homogenate (S9 fraction): 10 % v/v. The mean number of revertant colonies and sample standard deviation (from control plates) were calculated for each test group. A test compound was considered to be mutagenic when the following criteria were satisfied: i) the number of revertant colonies in any strain increased in the presence of one or more dose of the test compound, with or without metabolic activation ii) there was a dose-related increase in the number of revertant colonies, and iii) any increase was reproducible.

For both 5-fluoro-3-methoxy-2-pyrazinamine and 6-fluoro-3-methoxy-2-pyrazinamine the test result was negative indicating the compounds are not mutagenic in the test conditions.

## Claims

1. A compound of formula (I) or a pharmaceutically acceptable salt thereof:

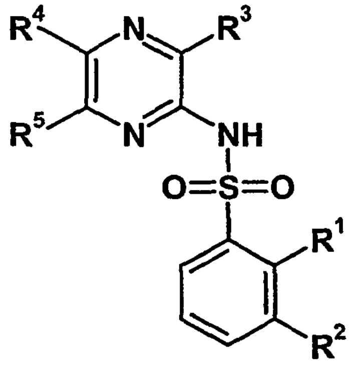

(I)

wherein
$R^1$ is selected from methyl, chlorine and fluorine;
$R^2$ is selected from methyl, chlorine and fluorine;
$R^3$ is methoxy;
one of $R^4$ and $R^5$ is fluorine and the other of $R^4$ and $R^5$ is selected from hydrogen and hydroxymethyl.

2. A compound according to claim 1, or a pharmaceutically acceptable salt thereof, wherein $R^1$ is selected from chlorine and fluorine, and $R^2$ is selected from chlorine and fluorine.

3. A compound according to claim 1 or claim 2, or a pharmaceutically acceptable salt thereof, wherein $R^4$ is fluorine and $R^5$ is selected from hydrogen and hydroxymethyl.

4. A compound according to claim 1 or claim 2, or a pharmaceutically acceptable salt thereof, wherein $R^5$ is fluorine and $R^4$ is selected from hydrogen and hydroxymethyl.

5. A compound according to claim 1 or claim 2, or a pharmaceutically acceptable salt thereof, wherein one of $R^4$ and $R^5$ is fluorine and the other of $R^4$ and $R^5$ is hydrogen.

6. A compound according to claim 1 or claim 2, or a pharmaceutically acceptable salt thereof, wherein one of $R^4$ and $R^5$ is fluorine and the other of $R^4$ and $R^5$ is hydroxymethyl.

7. A compound according to claim 1, which is selected from 2-Chloro-3-fluoro-*N*-(5-fluoro-3-methoxypyrazin-2-yl)-benzenesulfonamide, 2,3-Dichloro-*N*-(5-fluoro-3-methoxypyrazin-2-yl)-benzenesulfonamide, 2,3-Dichloro-*N*-(6-fluoro-3-methoxypyrazin-2-yl)-benzenesulfonamide, 2,3-Dichloro-*N*-[6-fluoro-5-(hydroxymethyl)-3-methoxypyrazin-2-yl]-benzenesulfonamide, 3-Chloro-2-fluoro-*N*-(5-fluoro-3-methoxypyrazin-2-yl)-benzenesulfonamide, 2,3-Dichloro-*N*-[5-fluoro-6-(hydroxymethyl)-3-methoxypyrazin-2-yl]-benzenesulfonamide, 3-Chloro-*N*-(5-fluoro-3-methoxypyrazin-2-yl)-2-methyl-benzenesulfonamide or a pharmaceutically acceptable salt thereof.

**8.** A compound according to claim 1, which is 2-Chloro-3-fluoro-*N*-(5-fluoro-3-methoxypyrazin-2-yl)- benzenesulfonamide or a pharmaceutically acceptable salt thereof.

**9.** A compound according to claim 1, which is 2,3-Dichloro-*N*-(5-fluoro-3-methoxypyrazin-2-yl)-benzenesulfonamide or a pharmaceutically acceptable salt thereof.

**10.** A pharmaceutical composition comprising a compound of formula (I), or a pharmaceutically acceptable salt thereof, as claimed in any one of claims 1 to 9 in association with a pharmaceutically acceptable adjuvant, diluent or carrier.

**11.** A compound of formula (I), or a pharmaceutically acceptable salt thereof, as claimed in any one of claims 1 to 9 for use in therapy.

**12.** Use of a compound of formula (I), or a pharmaceutically acceptable salt thereof, as claimed in any one of claims 1 to 9, in the manufacture of a medicament for use in the treatment of asthma.

**13.** A compound of formula (I), or a pharmaceutically acceptable salt thereof, as claimed in any one of claims 1 to 9, for treatment of asthma.

**14.** A process for the preparation of a compound of formula (I) or a pharmaceutically acceptable salt thereof, which comprises

(a) reacting a compound of formula (II), wherein $R^1$, $R^2$ and $R^3$ are as defined in formula (I) and one of $R^6$ and $R^7$ is hydrogen and the other of $R^6$ and $R^7$ is $NH_2$, with a nitrite salt in the presence of a fluorinating agent,

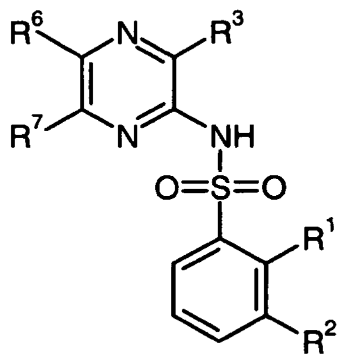

(II),

or

(b) reacting a compound of formula (III), wherein $R^1$, $R^2$ and $R^3$ are as defined in formula (I) and one of $R^8$ and $R^9$ is fluorine and the other of $R^8$ and $R^9$ is bromine, with hydrogen in the presence of a palladium catalyst,

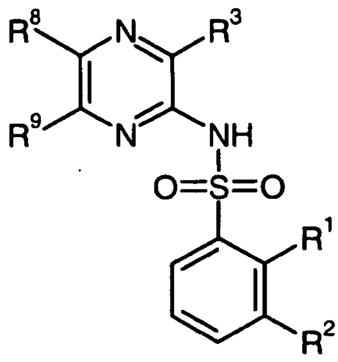

(III),

or

(c) where one of $R^4$ and $R^5$ is hydroxymethyl, reacting a compound of formula (III) as described in (b), with carbon monoxide in the presence of a palladium catalyst, and subsequently treating the resulting acid (or $C_{1-4}$ alkyl ester thereof) with a suitable reducing agent, or

(d) where one of $R^4$ and $R^5$ is fluorine and the other of $R^4$ and $R^5$ is hydrogen, reacting a compound of formula (IV), wherein $R^3$ is as defined in formula (I) and where one of $R^{10}$ and $R^{11}$ is fluorine and the other of $R^{10}$ and $R^{11}$ is hydrogen,

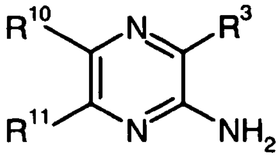

(IV),

with a compound of formula (V), wherein $R^1$ and $R^2$ are as defined in formula (I)

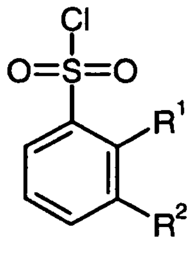

(V)

or

(e) where $R^2$ is fluorine and $R^1$ is chlorine, reacting a compound of formula (VI) wherein $R^3$, $R^4$ and $R^5$ are as defined in formula (I), with hexachloroethane in the presence of a lithium amide or alkyl lithium base,

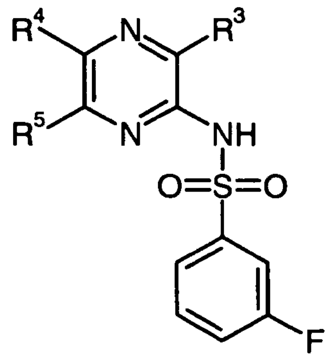

(VI),

and optionally after (a), (b), (c), (d) or (e) carrying out one or more of the following:

- converting the compound to a further compound of the invention or
- forming a pharmaceutically acceptable salt of the compound.

**15.** A compound of formula (IV),

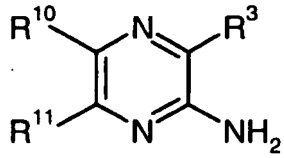

(IV)

wherein $R^3$ is methoxy; one of $R^{10}$ and $R^{11}$ is fluorine and the other of $R^{10}$ and $R^{11}$ is hydrogen.

**Patentansprüche**

**1.** Verbindungen der Formel (I) und deren pharmazeutisch annehmbare Salze:

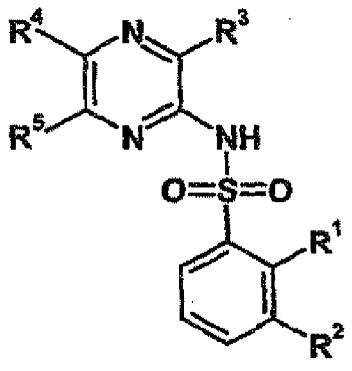

(I)

wobei
$R^1$ aus Methyl, Chlor und Fluor ausgewählt ist;
$R^2$ aus Methyl, Chlor und Fluor ausgewählt ist;
$R^3$ für Methoxy steht;
einer der Reste $R^4$ und $R^5$ für Fluor steht und der andere der Reste $R^4$ und $R^5$ aus Wasserstoff und Hydroxymethyl

ausgewählt ist.

2. Verbindungen nach Anspruch 1 und deren pharmazeutisch annehmbare Salze, wobei $R^1$ aus Chlor und Fluor ausgewählt ist und $R^2$ aus Chlor und Fluor ausgewählt ist.

3. Verbindungen nach Anspruch 1 oder 2 und deren pharmazeutisch annehmbare Salze, wobei $R^4$ für Fluor steht und $R^5$ aus Wasserstoff und Hydroxymethyl ausgewählt ist.

4. Verbindungen nach Anspruch 1 oder 2 und deren pharmazeutisch annehmbare Salze, wobei $R^5$ für Fluor steht und $R^4$ aus Wasserstoff und Hydroxymethyl ausgewählt ist.

5. Verbindungen nach Anspruch 1 oder 2 und deren pharmazeutisch annehmbare Salze, wobei einer der Reste $R^4$ und $R^5$ für Fluor steht und der andere der Reste $R^4$ und $R^5$ für Wasserstoff steht.

6. Verbindungen nach Anspruch 1 oder 2 und deren pharmazeutisch annehmbare Salze, wobei einer der Reste $R^4$ und $R^5$ für Fluor steht und der andere der Reste $R^4$ und $R^5$ für Hydroxymethyl steht.

7. Verbindungen nach Anspruch 1, ausgewählt aus
2-Chlor-3-fluor-*N*-(5-fluor-3-methoxypyrazin-2-yl)benzolsulfonsäureamid,
2,3-Dichlor-*N*-(5-fluor-3-methoxypyrazin-2-yl)benzolsulfonsäureamid,
2,3-Dichlor-*N*-(6-fluor-3-methoxypyrazin-2-yl)benzolsulfonsäureamid,
2,3-Dichlor-*N*-[6-fluor-5(hydroxymethyl)-3-methoxypyrazin-2-yl]benzolsulfonsäureamid,
3-Chlor-2-fluor-*N*-(5-fluor-3-methoxypyrazin-2-yl)benzolsulfonsäureamid,
2,3-Dichlor-*N*-[5-fluor-6-(hydroxymethyl)-3-methoxypyrazin-2-yl]benzolsulfonsäureamid,
3-Chlor-*N*-(5-Fluor-3-methoxypyrazin-2-yl)-2-methylbenzolsulfonsäureamid
und deren pharmazeutisch annehmbaren Salzen.

8. Verbindung nach Anspruch 1, bei der es sich um 2-Chlor-3-fluor-*N*-(5-fluor-3-methoxypyrazin-2-yl)benzolsulfonsäureamid oder ein pharmazeutisch annehmbares Salz davon handelt.

9. Verbindung nach Anspruch 1, bei der es sich um 2,3-Dichlor-*N*-(5-fluor-3-methoxypyrazin-2-yl)benzolsulfonsäureamid oder ein pharmazeutisch annehmbares Salz davon handelt.

10. Pharmazeutische Zusammensetzung, enthaltend eine Verbindung der Formel (I) oder ein pharmazeutisch annehmbares Salz davon nach einem der Ansprüche 1 bis 9 zusammen mit einem pharmazeutisch annehmbaren Adjuvans, Verdünnungsmittel oder Träger.

11. Verbindungen der Formel (I) und deren pharmazeutisch annehmbare Salze nach einem der Ansprüche 1 bis 9 zur Verwendung in der Therapie.

12. Verwendung einer Verbindung der Formel (I) oder eines pharmazeutisch annehmbaren Salzes davon nach einem der Ansprüche 1 bis 9 bei der Herstellung eines Medikaments zur Verwendung bei der Behandlung von Asthma.

13. Verbindungen der Formel (I) und deren pharmazeutisch annehmbare Salze nach einem der Ansprüche 1 bis 9 zur Behandlung von Asthma.

14. Verfahren zur Herstellung einer Verbindung der Formel (I) oder eines pharmazeutisch annehmbaren Salzes davon, bei dem man

(a) eine Verbindung der Formel (II), in welcher $R^1$, $R^2$ und $R^3$ wie in Formel (I) definiert sind und einer der Reste $R^6$ und $R^7$ für Wasserstoff und der andere der Reste $R^6$ und $R^7$ für $NH_2$ steht, in Gegenwart eines Fluorierungsmittels mit einem Nitritsalz umsetzt,

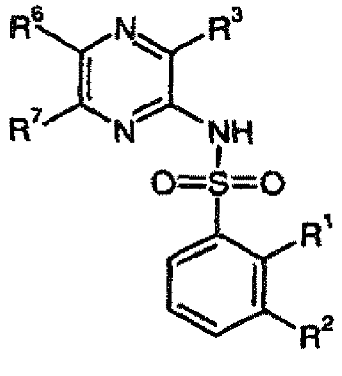

(II)

oder

(b) eine Verbindung der Formel (III), in welcher $R^1$, $R^2$ und $R^3$ wie in Formel (I) definiert sind und einer der Reste $R^8$ und $R^9$ für Fluor steht und der andere der Reste $R^8$ und $R^9$ für Brom steht, in Gegenwart eines Palladiumkatalysators mit Wasserstoff umsetzt,

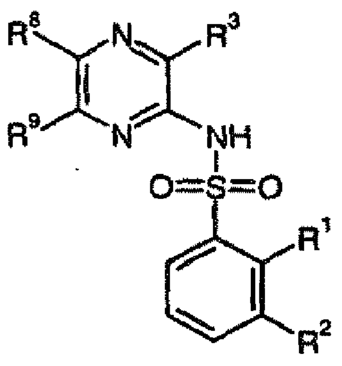

(III)

oder

(c) wenn einer der Reste $R^4$ und $R^5$ für Hydroxymethyl steht, eine wie in (b) beschriebene Verbindung der Formel (III) in Gegenwart eines Palladiumkatalysators mit Kohlenstoffmonoxid umsetzt und anschließend die erhaltene Säure (oder den $C_{1-4}$-Alkylester davon) mit einem geeigneten Reduktionsmittel behandelt, oder

(d) wenn einer der Reste $R^4$ und $R^5$ für Fluor steht und der andere der Reste $R^4$ und $R^5$ für Wasserstoff steht, eine Verbindung der Formel (IV), in welcher $R^3$ wie in Formel (I) definiert ist und wobei einer der Reste $R^{10}$ und $R^{11}$ für Fluor steht und der andere der Reste $R^{10}$ und $R^{11}$ für Wasserstoff steht,

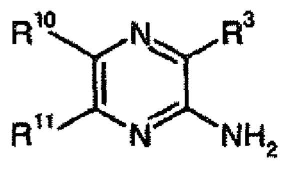

(IV)

mit einer Verbindung der Formel (V), in welcher $R^1$ und $R^2$ wie in Formel (I) definiert sind,

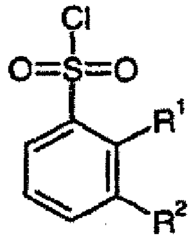

(V)

umsetzt, oder

(e) wenn $R^2$ für Fluor steht und $R^1$ für Chlor steht, eine Verbindung der Formel (VI), in welcher $R^3$, $R^4$ und $R^5$ wie in Formel (I) definiert sind, in Gegenwart eines Lithiumamids oder einer Alkyllithiumbase mit Hexachlorethan umsetzt,

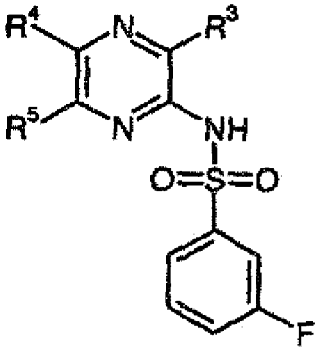

(VI)

und gegebenenfalls nach (a), (b), (c), (d) oder (e) einen oder mehrere der folgenden Schritte durchführt:

- Umwandlung der Verbindung in eine weitere erfindungsgemäße Verbindung oder
- Bilden eines pharmazeutisch annehmbaren Salzes der Verbindung.

**15.** Verbindungen der Formel (IV)

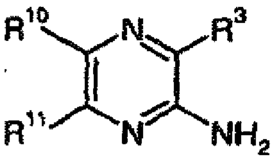

(IV)

in welcher $R^3$ für Methoxy steht, einer der Reste $R^{10}$ und $R^{11}$ für Fluor steht und der andere der Reste $R^{10}$ und $R^{11}$ für Wasserstoff steht.

**Revendications**

**1.** Composé de formule (I) ou un sel pharmaceutiquement acceptable de celui-ci :

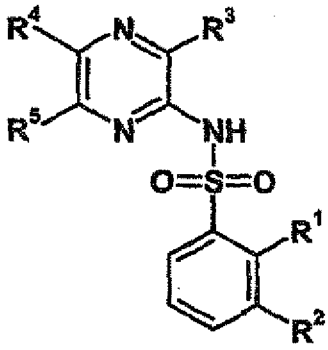

(I)

dans laquelle
$R^1$ est choisi parmi méthyle, chlore et fluor ;
$R^2$ est choisi parmi méthyle, chlore et fluor ;
$R^3$ est méthoxy ;
l'un de $R^4$ et $R^5$ est fluor et l'autre de $R^4$ et $R^5$ est choisi parmi hydrogène et hydroxyméthyle.

**2.** Composé selon la revendication 1, ou un sel pharmaceutiquement acceptable de celui-ci, **caractérisé en ce que** $R^1$ est choisi parmi chlore et fluor, et $R^2$ est choisi parmi chlore et fluor.

**3.** Composé selon la revendication 1 ou la revendication 2, ou un sel pharmaceutiquement acceptable de celui-ci, **caractérisé en ce que** $R^4$ est fluor, et $R^5$ est choisi parmi hydrogène et hydroxyméthyle.

**4.** Composé selon la revendication 1 ou la revendication 2, ou un sel pharmaceutiquement acceptable de celui-ci, **caractérisé en ce que** $R^5$ est fluor, et $R^4$ est choisi parmi hydrogène et hydroxyméthyle.

**5.** Composé selon la revendication 1 ou la revendication 2, ou un sel pharmaceutiquement acceptable de celui-ci, **caractérisé en ce que** l'un de $R^4$ et $R^5$ est fluor et l'autre de $R^4$ et $R^5$ est hydrogène.

**6.** Composé selon la revendication 1 ou la revendication 2, ou un sel pharmaceutiquement acceptable de celui-ci, **caractérisé en ce que** l'un de $R^4$ et $R^5$ est fluor et l'autre de $R^4$ et $R^5$ est hydroxyméthyle.

**7.** Composé selon la revendication 1, **caractérisé en ce qu'**il est choisi parmi
le 2-chloro-3-fluoro-N-(5-fluoro-3-méthoxypyrazin-2-yl)-benzènesulfonamide,
le 2,3-dichloro-*N*-(5-fluoro-3-méthoxypyrazin-2-yl)-benzènesulfonamide,
le 2,3-dichloro-*N*-(6-fluoro-3-méthoxypyrazin-2-yl)-benzènesulfonamide,
le 2,3-dichloro-*N*-[6-fluoro-5-(hydroxyméthyl)-3-méthoxypyrazin-2-yl]-benzènesulfonamide,
le 3-chloro-2-fluoro-*N*-(5-fluoro-3-méthoxypyrazin-2-yl)-benzènesulfonamide,
le 2,3-dichloro-*N*-[5-fluoro-6-(hydroxyméthyl)-3-méthoxypyrazin-2-yl]-benzènesulfonamide,
le 3-chloro-*N*-(5-fluoro-3-méthoxypyrazin-2-yl)-2-methyl-benzènesulfonamide ou un sel pharmaceutiquement acceptable de ceux-ci.

**8.** Composé selon la revendication 1, **caractérisé en ce qu'**il s'agit de 2-chloro-3-fluoro-N-(5-fluoro-3-méthoxypyrazin-2-yl)-benzènesulfonamide ou un sel pharmaceutiquement acceptable de celui-ci.

**9.** Composé selon la revendication 1, **caractérisé en ce qu'**il s'agit de 2,3-dichloro-N-(5-fluoro-3-méthoxypyrazin-2-yl)-benzènesulfonamide ou un sel pharmaceutiquement acceptable de celui-ci.

**10.** Composition pharmaceutique comprenant un composé de formule (I), ou un sel pharmaceutiquement acceptable de celui-ci, selon l'une quelconque des revendications 1 à 9 en association avec un adjuvant, un diluant ou un support pharmaceutiquement acceptable.

**11.** Composé de formule (I), ou un sel pharmaceutiquement acceptable de celui-ci, selon l'une quelconque des revendications 1 à 9, destiné à être utilisé en thérapie.

**12.** Utilisation d'un composé de formule (I), ou d'un sel pharmaceutiquement acceptable de celui-ci, selon l'une quelconque des revendications 1 à 9, dans la fabrication d'un médicament destiné à être utilisé dans le traitement de l'asthme.

**13.** Composé de formule (I), ou un sel pharmaceutiquement acceptable de celui-ci, selon l'une quelconque des revendications 1 à 9, destiné au traitement de l'asthme.

**14.** Procédé de préparation d'un composé de formule (I) ou d'un sel pharmaceutiquement acceptable de celui-ci, **caractérisé en ce qu'**il comprend

(a) la réaction d'un composé de formule (II), dans laquelle $R^1$, $R^2$ et $R^3$ sont tels que définis dans la formule (I) et l'un de $R^6$ et $R^7$ est hydrogène et l'autre de $R^6$ et $R^7$ est $NH_2$, avec un sel nitrite en présence d'un agent de fluoration,

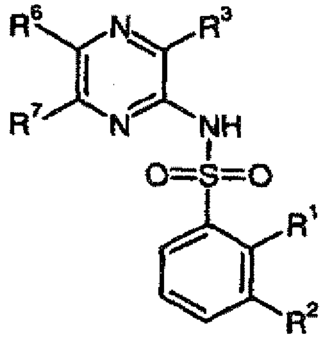
(II),

ou

(b) la réaction d'un composé de formule (III), dans laquelle $R^1$, $R^2$ et $R^3$ sont tels que définis dans la formule (I) et l'un de $R^8$ et $R^9$ est fluor et l'autre de $R^8$ et $R^9$ est brome, avec de l'hydrogène en présence d'un catalyseur de palladium,

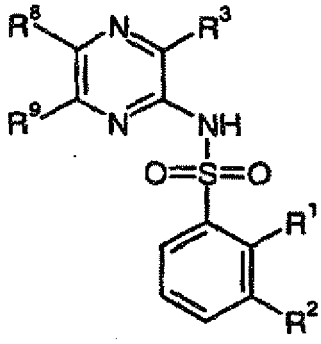
(III),

ou

(c) lorsque l'un de $R^4$ et $R^5$ est hydroxyméthyle, la réaction d'un composé de formule (III) tel que décrit dans (b), avec du monoxyde de carbone en présence d'un catalyseur de palladium, et ensuite le traitement de l'acide résultant (ou de l'ester d'alkyle en $C_{1-4}$ de celui-ci) avec un agent de réduction convenable, ou

(d) lorsque l'un de $R^4$ et $R^5$ est fluor et l'autre de $R^4$ et $R^5$ est hydrogène, la réaction d'un composé de formule (IV), dans laquelle $R^3$ est tel que défini dans la formule (I) et lorsque l'un de $R^{10}$ et $R^{11}$ est fluor et l'autre de $R^{10}$ et $R^{11}$ est hydrogène,

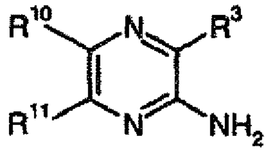

(IV),

avec un composé de formule (V), dans laquelle $R^1$ et $R^2$ sont tels que définis dans la formule (I)

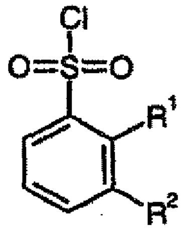

(V)

ou

(e) lorsque $R^2$ est fluor et $R^1$ est chlore, la réaction d'un composé de formule (VI) dans laquelle $R^3$, $R^4$ et $R^5$ sont tels que définis dans la formule (I), avec de l'hexachloroéthane en présence d'une base amide de lithium ou alkyllithium,

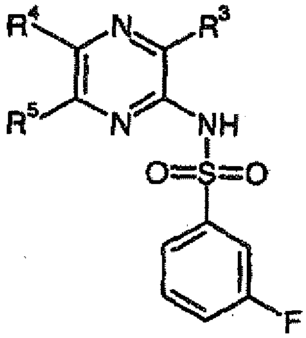

(VI),

et éventuellement après (a), (b), (c), (d) ou (e), la mise en oeuvre de l'une ou l'autre des étapes suivantes :

- la transformation du composé en un autre composé de l'invention ou
- la formation d'un sel pharmaceutiquement acceptable du composé.

**15.** Composé de formule (IV),

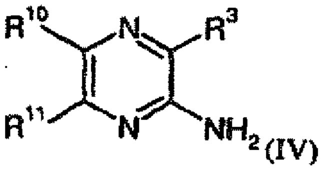

(IV)

dans laquelle $R^3$ est méthoxy ; l'un de $R^{10}$ et $R^{11}$ est fluor et l'autre de $R^{10}$ et $R^{11}$ est hydrogène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03051870 A
- WO 03059893 A
- WO 9722596 A
- WO 9730035 A
- WO 9732856 A
- WO 9813354 A
- WO 9902166 A
- WO 0040529 A
- WO 0041669 A
- WO 0192224 A
- WO 0204434 A
- WO 0208213 A
- WO 2003059893 A


### Non-patent literature cited in the description


- **Maron ; Ames.** *Mutation Res.,* 1983, vol. 113, 173-215
- **T.W. Greene ; P.G.M. Wuts.** Protective Groups in Organic Synthesis. Wiley-Interscience, 1991
- **P.J. Kocienski.** Protecting Groups. Georg Thieme Verlag, 1994
- *Synthesis,* 1990, 659-660